# EUROPEAN PATENT SPECIFICATION

(11) **EP 1 720 569 B1**
(45) Date of publication and mention of the grant of the patent: **29.10.2014**
(21) Application number: 05706314.1
(22) Date of filing: 28.02.2005
(51) Int. Cl.: A61K 39/395, G01N 33/532, C07K 16/30, C07K 16/42

(54) **TARGET FOR B-CELL DISORDERS**
TARGET FÜR B-ZELL-STÖRUNGEN
CIBLE POUR TROUBLES DES LYMPHOCYTES B

(30) Priority: 27.02.2004 US 548118 P
(43) Date of publication of application: 15.11.2006
(73) Proprietor: Immune System Therapeutics Ltd, Ultimo NSW 2007 (AU)
(72) Inventor: DUNN, Rosanne, Dorothy, Broadway, NSW 2007-03-21 (AU); JONES, Darren, Ross, Avalon, NSW 2107 (AU); ASVADI, Parisa, Lakemba, NSW 2195 (AU); RAISON, Robert, Pennant Hills, NSW 2120 (AU); HUTCHINSON, Andrew, Tasman, Chatswood, NSW 2067 (AU)
(74) Representative: Walcher, Armin
(86) International application number: PCT/AU2005/000280
(87) International publication number: WO 2005/082409

(56) References cited:
- EP-A- 1 342 779
- WO-A-03/004056
- NAKANO T ET AL: "ELISAs for free light chains of human immunoglobulins using monoclonal antibodies: comparison of their specificity with available polyclonal antibodies" JOURNAL OF IMMUNOLOGICAL METHODS, ELSEVIER SCIENCE PUBLISHERS B.V.,AMSTERDAM, NL, vol. 275, no. 1-2, 1 April 2003 (2003-04-01), pages 9-17, XP004416746 ISSN: 0022-1759
- WALKER K Z ET AL: "A MONOCLONAL ANTIBODY WITH SELECTIVITY FOR HUMAN KAPPA MYELOMA AND LYMPHOMA CELLS WHICH HAS POTENTIAL AS A THERAPEUTIC AGENT" ADVANCES IN EXPERIMENTAL MEDICINE AND BIOLOGY, SPRING ST., NY, US, vol. 186, 1985, pages 833-841, XP008030775 ISSN: 0065-2598
- DUNN R D ET AL: "Antigen binding and cytotoxic properties of a recombinant immunotoxin incorporating the lytic peptide, melittin" IMMUNOTECHNOLOGY, ELSEVIER SCIENCE PUBLISHERS BV, NL, vol. 2, no. 3, September 1996 (1996-09), pages 229-240, XP004070297 ISSN: 1380-2933
- ABE M. ET AL: 'Production and immunodiagnostic applications of antihuman light chain monoclonal antibodies.' AMERICAN JOURNAL OF CLINICAL PATHOLOGY. vol. 100, no. 1, 1993, pages 67 - 74, XP008074229
- BOUX H.A. ET AL: 'A tumor-associated antigen specific for human kappa myeloma cells.' JOURNAL OF EXPERIMENTAL MEDICINE. vol. 158, no. 5, 1983, pages 1769 - 1774, XP001191239
- WALKER K.Z. ET AL: 'A rat model system for radioimmunodetection of kappa myeloma antigen on malignant B cells.' EUROPEAN JOURNAL OF NUCLEAR MEDICINE. vol. 12, no. 9, 1986, pages 461 - 467, XP008030786
- LAUDER I. ET AL: 'Surface membrane phenotypic expression and treatment response of malignant lymphomas.' JOURNAL OF PATHOLOGY. vol. 145, no. 3, 1985, pages 259 - 268, XP008074230
- ARPIN C. ET AL: 'The normal counterpart of IgD myeloma cells in germinal center displays extensive mutated IgVH gene, CMU-Cdelta switch, and lambda light chain expression.' JOURNAL OF EXPERIMENTAL MEDICINE. vol. 187, no. 8, 1998, pages 1169 - 1178, XP008074225
- BRADWELL A.R. ET AL: 'Highly sensitive. automated immunoassay for immunoglobulin free light chains in serum and urine.' CLINICAL CHEMISTRY. vol. 47, no. 4, 2001, pages 673 - 680, XP008063172
- TILLYER C.R. ET AL: 'Immunoturbidimetric assay for estimating free light chains of immunoglobulin in urine and serum.' JIURNAL OF CLINICAL PATHOLOGY. vol. 44, no. 6, 1991, pages 466 - 471, XP008074231
- BERGSAGEL P.L. ET AL: 'In multiple myeloma, clonotypic B lymphocytes are detectable among CD19+ peripheral blood cells expressing CD38, CD56, and monotypic Ig light chains.' BLOOD. vol. 75, no. 2, 1995, pages 436 - 447, XP008074224
- BODEY B. ET AL: 'Genetically engineered monoclonal antibodies for direct antineoplastic treatment and cancer cell specific delivery of chemotherapeutic agents.' CURRENT PHARMACEUTICAL DESIGN. vol. 6, no. 3, 2000, pages 261 - 276, XP003003635
- TORDSSON J.M. ET AL: 'Phage-selected primate antibodies fused to superantigens for immunotherapy of malignant melanoma.' CANCER IMMUNOLOGY IMMUNOTHERAPY. vol. 48, no. 12, 2000, pages 691 - 702, XP008074238
- KOVAR M. ET AL: 'HPMA copolymer-bound doxorubucin targeted to tumor specific antigen of BCL1 mouse B cell leukemia.' JOURNAL OF CONTROLED RELEASE. vol. 92, no. 3, 2003, pages 315 - 330, XP004467645
- TUTT A L ET AL: "Antibodies against urinary light chain idiotypes as agents for detection and destruction of human neoplastic B lymphocytes.", JOURNAL OF IMMUNOLOGY (BALTIMORE, MD. : 1950) DEC 1983 LNKD- PUBMED:6417240, vol. 131, no. 6, December 1983 (1983-12), pages 3058-3063, ISSN: 0022-1767
- WALDMANN T A: "MONOCLONAL ANTIBODIES IN DIAGNOSIS AND THERAPY", SCIENCE, AMERICAN ASSOCIATION FOR THE ADVANCEMENT OF SCIENCE, WASHINGTON, DC; US, vol. 252, no. 5013, 21 June 1991 (1991-06-21), pages 1657-1661, XP000232755, ISSN: 0036-8075, DOI: 10.1126/SCIENCE.2047874
- FU S M ET AL: "Occurrence of surface IgM, IgD, and free light chains of human lymphocytes.", THE JOURNAL OF EXPERIMENTAL MEDICINE 1 FEB 1974 LNKD- PUBMED:4589993, vol. 139, no. 2, 1 February 1974 (1974-02-01), pages 451-456, ISSN: 0022-1007
- CESANA CLARA ET AL: "Prognostic factors for malignant transformation in monoclonal gammopathy of undetermined significance and smoldering multiple myeloma.", JOURNAL OF CLINICAL ONCOLOGY : OFFICIAL JOURNAL OF THE AMERICAN SOCIETY OF CLINICAL ONCOLOGY 15 MAR 2002 LNKD- PUBMED:11896113, vol. 20, no. 6, 15 March 2002 (2002-03-15) , pages 1625-1634, ISSN: 0732-183X
- SACHS JONATHAN N ET AL: "Introduction to the membrane protein reviews: the interplay of structure, dynamics, and environment in membrane protein function.", ANNUAL REVIEW OF BIOCHEMISTRY 2006 LNKD- PUBMED:16756508, vol. 75, 2006, pages 707-712, ISSN: 0066-4154
- SOLOMON AND WEISS: "Protein and host factors implicated in the pathogenesis of light chain amyloidosis", AMYLOID: INT. J. EXP. CLIN. INVEST., vol. 2, 1995, pages 269-279,
- KHURANA R ET AL: "Partially folded intermediates as critical precursors of light chain amyloid fibrils and amorphous aggregates", BIOCHEMISTRY 20010327 US LNKD- DOI:10.1021/BI001782B, vol. 40, no. 12, 27 March 2001 (2001-03-27), pages 3525-3535, ISSN: 0006-2960
- GRAILLE M ET AL: "Complex between Peptostreptococcus magnus protein L and a human antibody reveals structural convergence in the interaction modes of Fab binding proteins.", STRUCTURE (LONDON, ENGLAND : 1993) AUG 2001 LNKD- PUBMED:11587642, vol. 9, no. 8, August 2001 (2001-08), pages 679-687, ISSN: 0969-2126
- KABAT E A ET AL: "Evolutionary and structural influences on light chain constant (CL) region of human and mouse immunoglobulins.", PROCEEDINGS OF THE NATIONAL ACADEMY OF SCIENCES OF THE UNITED STATES OF AMERICA JUL 1975 LNKD- PUBMED:809770, vol. 72, no. 7, July 1975 (1975-07), pages 2785-2788, ISSN: 0027-8424
- DARIAVACH P ET AL: "Human immunoglobulin C lambda 6 gene encodes the Kern+Oz-lambda chain and C lambda 4 and C lambda 5 are pseudogenes.", PROCEEDINGS OF THE NATIONAL ACADEMY OF SCIENCES OF THE UNITED STATES OF AMERICA DEC 1987 LNKD- PUBMED:3122211, vol. 84, no. 24, December 1987 (1987-12), pages 9074-9078, ISSN: 0027-8424
- ZIMMER F J ET AL: "Transposition of human immunoglobulin V kappa genes within the same chromosome and the mechanism of their amplification.", THE EMBO JOURNAL MAY 1990 LNKD- PUBMED:2109696, vol. 9, no. 5, May 1990 (1990-05), pages 1535-1542, ISSN: 0261-4189
- GORDON J ET AL: "Free light chain synthesis by neoplastic cells in chronic lymphocytic leukaemia and non-Hodgkin's lymphoma.", IMMUNOLOGY MAR 1978 LNKD- PUBMED:417018, vol. 34, no. 3, March 1978 (1978-03), pages 397-404, ISSN: 0019-2805

## Description

### Field of the Invention

The present disclosure relates to the diagnosis and treatment of B-cell disorders such as multiple myeloma (MM). In particular, the present invention relates to the treatment of lymphoproliferative disorders using ligands which bind to free lambda light chains expressed on the surface of lymphoid cells.

### Background of the invention

Multiple myeloma is a cancer of the blood in which the malignant cell is a terminally differentiated monoclonal B cell. Conventional treatment of this disease is a high dose chemotherapy regime with or without autologous stem cell transplantation. However, there is now overwhelming clinical evidence that this treatment regime will inevitably fail because the tumour will ultimately become refractory (Davies et al. (2000) Eur. J. Haematol. 64:359-367; Ryoo et al. (2002) Blood Rev. 16:167-174; Kyle RA, (2001a) Oncologist 6:119-124).

### Current Treatments for Multiple Myeloma

Current therapies for MM have been reported in the literature and include variations of high-dose chemotherapy (Kyle RA (2001a) Oncologist 6:119-124; Kyle RA (2001b) Seminars in Hematology 38; 2; 3; 11-14, Anderson et al. (1999) Seminars in Hematology 36; 1; 3-8). Most patients with MM have symptomatic disease at diagnosis and require therapy, however, some patients are asymptomatic and are generally not treated until they become symptomatic.

The treatment of choice for MM patients younger than 65 years is autologous peripheral blood stem cell transplantation (APBST) in combination with chemotherapy (Harousseau and Attal (2002) Blood Reviews 16; 245-253). Chemotherapy alone is the preferred initial treatment for patients older than 65 years or younger patients for whom transplantation is not feasible. APBST is applicable for more than half of patients with MM. Despite attempts to reduce tumour cell contamination of the grafts it has been shown that autologous peripheral stem cells are generally contaminated by myeloma cells or their precursors. This results in re-population of the bone marrow with malignant cells and ultimately in relapse.

Initial treatment for symptomatic MM patients is high-dose chemotherapy (Kyle RA, (2001a) The Oncologist 6; 2; 119-124; Kyle RA, (2001b) Seminars in Hematology 38; 2; 3; 11-14, Anderson et al. (1999) Seminars in Hematology 36; 1; 3-8). Most physicians treat the patients with vincristine, doxorubicin (Adriamycin) and dexamethasone (VAD) for 3-4 months. This results in a reduction of tumour cells in the bone marrow and peripheral blood. High dose cyclophosphamide and granulocyte-colony stimulating factor (G-CSF) are then administered. G-CSF stimulates the production of peripheral stem cells (CD34+ B-cells) for autologous peripheral blood stem cell transplantation. At this stage peripheral blood is taken and stem cells are collected using a fluorescence activated cell sorter (FACS).

Currently there is a choice of at least two treatment regimes prior to bone marrow engraftment with stem cells. In the first case the patient can be given high-dose chemotherapy and/or total body irradiation followed by APBST. Alternatively the patient can be given alkylating agents after stem cell collection until a plateau is reached. Then α₂-interferon can be given to inhibit cell growth and division, or no therapy can be given until relapse. At this stage the patient is given high-dose melphalan and/or total body irradiation and the previously collected blood stem cells infused. In general, melphalan (200 mg/m²) is given as it is less toxic and there appears to be no advantage in using total body irradiation.

Comparative studies of conventional chemotherapy and high dose chemotherapy in combination with APBST in MM patients indicated that the latter significantly improved the event free survival and overall survival (Harousseau and Attal (2002) Blood Reviews 16; 245-253). At present VAD treatment followed by APBST results in a favourable five-year survival rate in the transplantation group versus VAD treatment alone (52% vs 12%). Chemotherapy can be continued until the patient is in a plateau state or for one year. If relapse occurs in the plateau stage after 6 months the chemotherapy regime should be re-instituted. Despite the recent improvements in treatment regimes, long-term follow up of these clinical studies has shown that elimination of myeloma from the patient does not occur even with large doses of chemotherapy and APBST.

Thus the current treatment extends the life of the patient but is not curative. It is now apparent that disease progression is associated with genetic instability and more specifically with dysregulation of genes involved in adhesion, apoptosis, cell cycle, drug resistance, growth arrest, oncogenesis, signaling and transcription (Zhan et al. (2002) Blood 99; 5; 1745-1757).

### Disease Progression is a Multistep Transformation Process

Several studies have suggested that disease progression in multiple myeloma correlates with progressive genetic events in the malignant plasma cell (Hallek et al. (1998) Blood 91, 1; 3-21; Avet-Loiseau et al. (2002) Blood 99; 6; 2185-2191; Zhan et al. (2002) Blood 99; 5; 1745-1757). The progressive stages of the disease appear to be initiated by a pre-existing monoclonal plasma cell disorder referred to as monoclonal gammopathy of undetermined significance (MGUS) where the cells are immortalized, but not transformed. The next stage of progression is intramedullary myeloma where the cells are found only in the bone marrow and are dependent on bone marrow stromal cells (BMSCs) for survival. In particular, a paracrine loop for interleukin-6 (IL-6) and the interleukin-6 receptor (IL-6R) develops between MM cells and the BMSCs. IL-6 appears to be the most important cytokine in establishing myeloma cells in the bone marrow. The most marked effect is the ability of IL-6 to inhibit dexamethasone-induced apoptosis in myeloma cells.

Concomitantly, myeloma cells stimulate osteoclasts that are responsible for bone resorption resulting in the characteristic bone lesions found in MM. Following this stage is an extra-medullary phase where the cells proliferate more rapidly and grow in the blood (plasma cell leukaemia, PCL) or other extra-medullary sites. The final stage of development is where cells become completely dysregulated and may be propagated *in vitro.*

### Generic Abnormalities

Disease progression at the cellular level is closely linked to genetic abnormalities that are associated with specific examples of gene dysregulation (Hallek et al. (1998) Blood 91, 1; 3-21). Several karyotypic studies have shown that aneuploidy is a common characteristic of myeloma cells and is independent of disease stage. A review of these studies has suggested that there are two major categories of genetic abnormalities in multiple myeloma (Fonseca et al. (2004) Cancer Research 64; 1546-1558). One category consists of patients with translocations involving the immunoglobulin heavy chain locus (IgH) which accounts for approximately half the genetic abnormalities in myeloma patients. It is also clear that the hypodiploid karyotypes and chromosome 13 monosomy are commonly associated with IgH translocations. The prevalence and clinical importance of specific IgH translocations have recently been determined and are reported in Fonseca et al. (2004) Cancer Research 64; 1546-1558,

The remaining 50% of patients appear to have the hyperdiploid karyotype and do not have IgH translocations.

Translocations that involve the light chain (*IgL*) genes have not been well characterised. One study has indicated that IgL-λ translocations are present in approximately 10% of MGUS samples and approximately 20% of intramedullary MM tumours (Fonseca et al. (2002) Blood, 100; 1417-1424). Translocations of IgL-κ have only been identified in a small number of tumours from intramedullary MM (Fonseca et al. (2004) Cancer Research 64; 1546-1558). At present, the clinical importance of IgL translocations is unknown.

### The monoclonal protein of myeloma cells

Despite the complexity of the karyotypic abnormalities found in multiple myeloma, a laboratory hallmark of this disease is the production and secretion of monoclonal protein (M-protein) into the blood and/or urine. In general the M-protein is an immunoglobulin, or a component of an immunoglobulin, that has not retained normal antibody function. Excess lambda or kappa light chains (Bence Jones proteins, BJP) are commonly produced by the myeloma cells. BJP's are present in the cytoplasm of the cells and are also secreted into the blood. They are frequently secreted as monomer (22-25 kD) and dimer (50 kD) forms and are small enough to pass freely into the urine (Durie (2003) International Myeloma Foundation, Multiple Myeloma, Concise Review of the Disease and Treatment Options).

The presence of membrane bound lambda light chain that is not associated with heavy chain would provide a novel therapeutic target on myeloma cells.

Non-patent literature Fu S.M. et. al (J.Exp.M. Vol. 139, 1974, pages 451 - 455) describes the occurence of surface IgM, IgD, and free light chains on human lymphocytes.

### Summary of the Invention

The present inventors have now identified a new target on myeloma cells for use in methods designed for the diagnosis or treatment of multiple myeloma. This target is free lambda light chain expressed on the surface of myeloma cells. By "free lambda light chain" we mean a lambda light chain that is not associated with an intact immunoglobulin. Free lambda light chain expressed on the surface of myeloma cells is referred to herein as lambda myeloma antigen ("LMA").

The therapeutic use proposed by the present inventors are based on the administration of a ligand that binds specifically to LMA for the depletion of malignant cells in subjects suffering from B-cell disorders. The ligand is an anti-LMA antibody. The therapeutic approaches described herein represent a radical departure from previous and currently available treatments for B-cell disorders.

The present invention provides the use of an anti-LMA antibody according to claim 1.

The B-cell disorder is a lymphoproliferative disorder.

The lymphoproliferative disorder is multiple myeloma, and more preferably lambda-type multiple myeloma.

The present invention can be used in a method for inhibiting the growth of or killing lymphoid cells in a subject, the method comprising administering to the subject an anti-LMA antibody under conditions sufficient for the binding of the anti-LMA antibody to the lymphoid cells to inhibit the growth of, or to kill, the cells. For example, the inhibition or killing of the lymphoid cells may be effected by apoptosis or by the immune cells of the subject (such as by antibody-dependent cell-mediated cytotoxicity (ADCC)).

The present invention also provides an anti-lambda myeloma antigen antibody according to claim 4.

According to the invention, the lymphoid cells are myeloma cells.

The term "LMA ligand conjugate" as used herein refers to an LMA ligand conjugated to a cytotoxic moiety or biological modifier.

The binding between a ligand and LMA be mediated by covalent or non-covalent interactions or a combination of covalent and non-covalent interactions. When the interaction of the ligand and LMA produces a non-covalently bound complex, the binding which occurs is typically electrostatic, hydrogen-bonding, or the result of hydrophilic/lipophilic interactions. LMA ligands are anti-LMA antibodies.

In one embodiment, the cytotoxic moiety is a toxin (which may be a photo-activated toxin), a chemotherapeutic agent, or a radioactive agent.

By way of non-limiting examples, the cytotoxic moiety may be a cytotoxic drug or an enzymatically active toxin of bacterial or plant origin (such as gelonin), or an enzymatically active fragment ("A chain") of such a toxin. Enzymatically active toxins and fragments thereof are preferred and are exemplified by gelonin, diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytoiacca americana proteins (PAPI, PAPII, and PAP-S), momordica charantia inhibitor, curcin, crotin, saponaria officinalis inhibitor, mitogellin, restrictocin, phenomycin, and enomycin.

Cytotoxic drugs which are useful in the present invention include, but are not limited to, adriamycin (and derivatives thereof), cis-platinum complex (and derivatives thereof), bleomycin and methotrexate (and derivatives thereof). These cytotoxic drugs are sometimes useful for clinical management of recurrent tumors and particularly breast cancer, but their use is complicated by severe side effects and damage caused to non-target cells. Anti-LMA antibodies may serve as a useful carrier of such drugs providing an efficient means of both delivery to the cancer cells and enhanced entry into the cancer cells themselves.

The cytotoxic moiety may also be a radioactive agent. In particular, the cytotoxic moiety may be a radionuclide such as, for example, Yttrium-90 (⁹⁰Y), Indium-111 (¹¹¹In), Iodine-131 (¹³¹I) or copper-67 (⁶⁷Cu).

Biological response modifiers which may be coupled to the LMA ligand and used in the present invention include, but are not limited to, lymphokines and cytokines such as IL-2 and interferons (α, β or γ). These biological response modifiers have a variety of effects on tumor cells. Among these effects are increased tumor cell killing by direct action as well as increased tumor cell killing by increased host defence mediated processes. Conjugation of an LMA ligand to these biological response modifiers will allow selective localization within lymphoid cells and, hence, improved antiproliferative effects while suppressing non-specific effects leading to toxicity of non-target cells.

Conjugates comprising LMA ligands may be made using a variety of bifunctional protein coupling agents. Examples of such reagents are SPDP, IT, bifunctional derivatives of imidoesters such as dimethyl adipimidate HCl, active esters such as disuccinimidyl suberate, aldehydes such as glutaraldehyde, bis-azido compounds such as bis(p-azidobenzoyl) hexanediamine, bis-diazonium derivatives such as bis-(p-diazoniumbenzoyl)-ethylenediamine, diisocyanates such as tolylene 2,6-diisocyanate, and bis-active fluorine compounds such as a 1,5-difluoro-2,4-dinitrobenzene.

In another embodiment the cytotoxic moiety is a nucleic acid molecule encoding a cytotoxic agent. In this embodiment, the LMA ligand functions as a carrier to introduce a therapeutic gene encoding a cytotoxic agent, e.g., toxin genes such as diphtheria toxin-A, lectins, Pseudomonas exotoxin A, Saponaria officinalis SO-6 (Soria M, (1989) Pharmacol. Res., 21 Suppl2:35-46) or ricin; cell suicide genes such as thynidine kinase or nitroreductase; proteins that activate chemotherapeutic genes such as gangcyclovir or mitomycin C; a ribozyme, RNase, or an antisense sequence (e.g., BCL2 sequence); into LMA+ cells such as myeloma cells. Preferably, the expression of only a few molecules of the cytotoxic agent encoded by the therapeutic gene are sufficient to kill a cell that expresses that gene. It is preferred that the therapeutic gene is operatively linked to a cell or tissue-specific transcription unit, e.g., a cell or tissue-specific promoter and/or enhancer. Preferred transcription units are those which direct expression in B cells (e.g., transcription units from an Ig heavy gene, Ig kappa gene, Ig lambda gene, BCL-6 gene (Dalla Favera et al., C.S.H. Smp. Quant. Biol., 59, 117 (1994)), CD19 gene, CD20 gene, or CD22 gene (Kerhl et al., Immunol. Today, 15,432 (1994)), T cells (e.g., transcription units from the IL-4 gene, IL-2 gene, IL-2R gene, T cell receptor gene, IL-5 gene, IL-13 gene, GM-CSF gene and Fas ligand gene (Nagata et al., Prog. Mol. Subcell. Biol., 16, 87 (1996)) or myeloid cells. Myeloid-specific transcription units include, but are not limited to, those disclosed in U.S. Pat. No. 5,502,176, as well as transcription units from the PU.1 gene (Fisher et al., Stem Cells, 16,25 (1998)), CD11c or CD18 gene (Corbi et al., Leuk. & Lymph., 25, 415 (1997)), IgH enhancer, CSF receptor G, GM and/or G gene (Zhang et al., Cur. Top. Micro. & Immunol., 211, 137 (1996)), or the C/EBP, Runt/PEBP2/CBF or Ets gene (Clarke et al., J. Leuko, Biol., 63, 153 (1998)).

In a preferred embodiment the subject has been treated to reduce the levels of free lambda light chains present in the fluid of the subject prior to administration of the anti-LMA antibody or LMA ligand conjugate. Preferably, the levels of free light chains present in the serum of the subject are reduced. A reduction in the levels of free light chains in the serum may be achieved by, for example, chemotherapy or plasmapheresis, or by methods where an LMA ligand (such as an anti-LMA antibody) is bound to a solid support (for example, a sterile filter) and used to capture free lambda light chains from the fluid. It is preferred that the treatment for reducing levels of free light chains in the fluid of the subject is performed on the subject just prior to administration of the anti-LMA antibody or LMA ligand conjugate.

The present invention can be used for removing lymphoid cells from an isolated cellular sample, such as, but not limited to, bone marrow cells, by exposing the cellular sample to an anti-LMA antibody or LMA ligand conjugate under conditions wherein the lymphoid cells bind to the antibody or conjugate, and isolating a cellular fraction of said cellular sample which does not bind to the antibody or conjugate. This may be used, for example, in the removal of myeloma cells from a bone marrow sample for autologous bone marrow transplant.

The present invention can be used in a method for autologous hematopoietic cell transplantation in a subject, the method comprising
(i) removing a hematopoietic progenitor cell population from the subject,
(ii) treating the cell population with an anti-LMA antibody or LMA ligand conjugate, and
(iii) transplanting the treated cell population from step (ii) into the subject.

The step of treating the progenitor cell population with an anti-LMA antibody or LMA ligand conjugate preferably involves contacting the cell population with an anti-LMA antibody or LMA ligand conjugate under conditions sufficient for the binding of the anti-LMA antibody or LMA conjugate to lymphoid cells present in the population to inhibit the growth of, or to kill, the lymphoid cells.

The method can involve intravenous infusion of anti-LMA antibody or LMA ligand conjugate into the subject.

The method of autologous transplantation can be performed on the subject during or after cytoreductive therapy.

The anti-LMA antibody or LMA ligand conjugate can be bound to a solid support.

The above-mentioned conjugate or the anti-LMA antibody may be used *in vitro* to inhibit growth of, or kill, lymphoid cells in a cellular sample, such as a bone marrow sample.

The invention can be used in a method provided for localizing lymphoid cells in a subject by administering an anti-LMA antibody, allowing the antibody to bind to cells within the subject, and determining the location of the antibody within the subject. The antibody can be detectably labeled, for example, with a radionuclide, a fluorophore, a chromophore or an enzyme.

The labels that may be used in making labeled versions of the antibodies include moieties that may be detected directly, such as fluorochromes and radiolabels as well as moieties, such as enzymes, that must be reacted or derivatized to be detected. Examples of such labels are ³²P, ¹²⁵I, ³H, ¹⁴C, fluorescein and its derivatives, rhodamine and its derivatives, dansyl, umbelliferone, luciferia, 2,3-dihydrophthalzainediones, horseradish peroxidase, alkaline phosphatase, lysozyme, and glucose-6-phosphate dehydrogenase. The antibodies may be tagged with such labels by known methods. For instance, coupling agents such as aldehydes, carbodiimides, dimaleimide, imidates, succinimides, bis-diazotized benzadine and the like may be used to couple the antibodies with the above-described fluorescent, chemiluminescent, and enzyme labels.

In another aspect the present invention provides an anti-LMA antibody conjugated to a cytotoxic moiety or a biological modifier. In one embodiment of this aspect, the cytotoxic moiety is a toxin, a photo-activated toxin, a chemotherapeutic agent, or a radioactive agent.

In another embodiment of this aspect, the cytotoxic moiety is a nucleic acid molecule. Thus, the invention provides a therapeutic composition which selectively targets LMA cell surface molecules but has reduced or no immunogenicity as the therapeutic gene, preferably in the form of circular DNA such as plasmid DNA, rather than an immunogenic protein, is introduced to the host mammal. As a result, it may be possible to repeatedly administer the therapeutic composition to a mammal, e.g., myeloma subjects, without the development of significant antibody responses, particularly to the cytotoxic agent encoded by the therapeutic gene. Moreover, a therapeutic composition of the invention is useful to kill cells in subjects with other LMA+ plasmaproliferative disorders such as B cell lymphoma and macroglobulinemia. Preferably, a humanized version of the antibody portion of the fusion polypeptide in the composition is employed for use in humans.

In another aspect, the present invention is directed to an anti-LMA antibody labeled with a detectable moiety, such as, by way of non-limiting examples, a fluorophore, a chromophore, a radionuclide, or an enzyme.

In still yet another aspect, the invention is directed to a pharmaceutical composition comprising an anti-LMA antibody of the invention and a pharmaceutically-acceptable carrier, diluent, or excipient.

The invention is also directed to an anti-LMA antibody or LMA ligand/cytotoxin bound to a solid support.

In a further preferred embodiment of the present invention, the anti-LMA antibody is a chimeric antibody or a humanised antibody.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The various features and embodiments of the present invention, referred to in individual sections above apply, as appropriate, to other sections, *mutatis mutandis.* Consequently features specified in one section may be combined with features specified in other sections, as appropriate.

### Brief description of the Figures

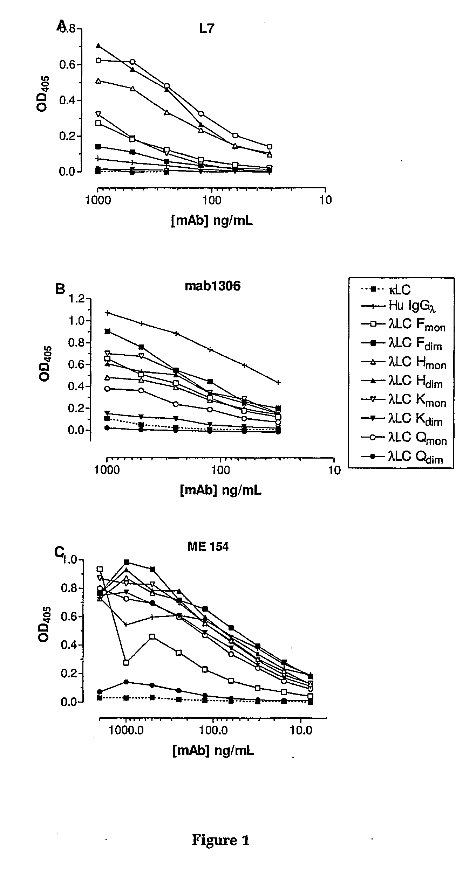

**Figure 1****.** Analysis of mAb binding to free lambda light chains by ELISA. The mAbs L7 (A), mab1306 (B) and ME 154 (C) were incubated against antigens consisting of human lambda light chain (λLC F, λLC H, λLC K and λLC Q in both monomeric (mon) and dimeric (dim) forms, pooled normal human IgGλ (Hu IgGλ), and free human kappa light chain (κLC) immobilised on ELISA plates. Bound mAbs were detected with an anti-mouse IgG-AP conjugate. Antibody-antigen complexes were visualized by pNPP enzyme reaction and absorbance measured at 405nm.

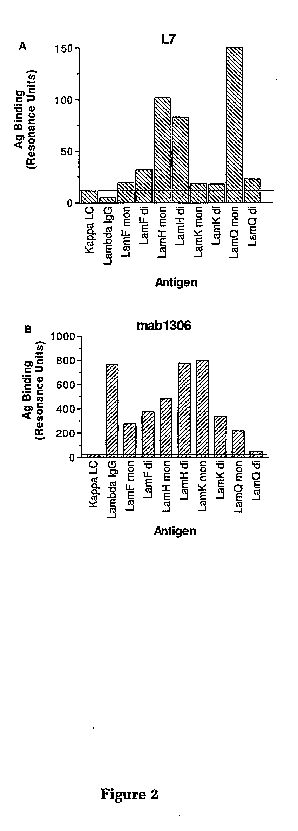

**Figure 2****.** Analysis of mAb binding to free lambda light chains by Surface Plasmon Resonance (SPR). The mAbs L7 (A) and mab1306 (B) were immobilised on a Biacore CM5-dextran chip. Antigens consisting of human lambda light chain (Lam F, Lam H, Lam K, Lam L, Lam Q in both monomeric (mon) and dimeric (dim) forms, pooled normal human IgGλ (Lambda IgG) and free human kappa light chain (Kappa LC) were passed over the immobilised mAbs. Antibody-antigen binding was measured by SPR using a Biacore 2000 Biosensor.

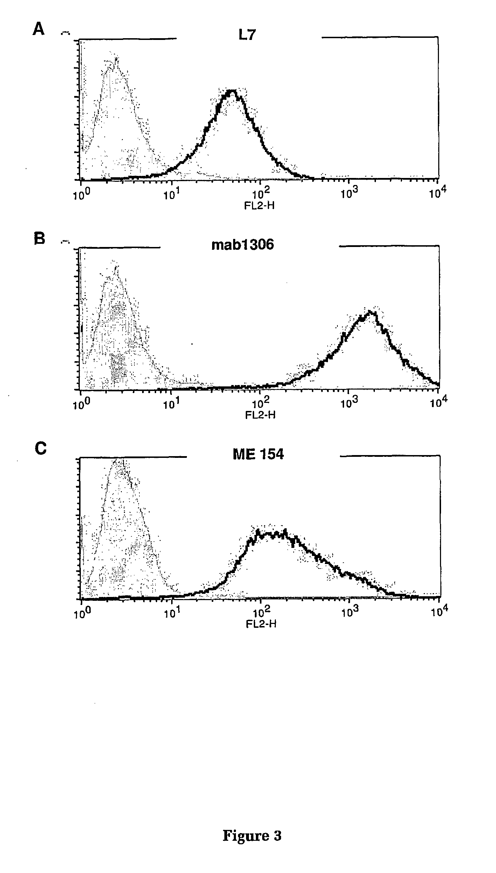

**Figure 3****.** Binding of anti-λ mAbs to LP-1 myeloma cells. LP-1 λ myeloma cells (5 x 10⁵) were incubated with 50 µL of 100µg/mL of mAb L7 (A), mab1306 (B) or ME 154 (C) for 30 minutes on ice. Cells were then washed twice and incubated with PE-labelled goat-anti-mouse F(ab')₂, washed and analysed by flow cytometry. The solid grey histogram represents cells which were incubated with an irrelevant mAb followed by the PE-labelled goat-anti-mouse F(ab')₂.

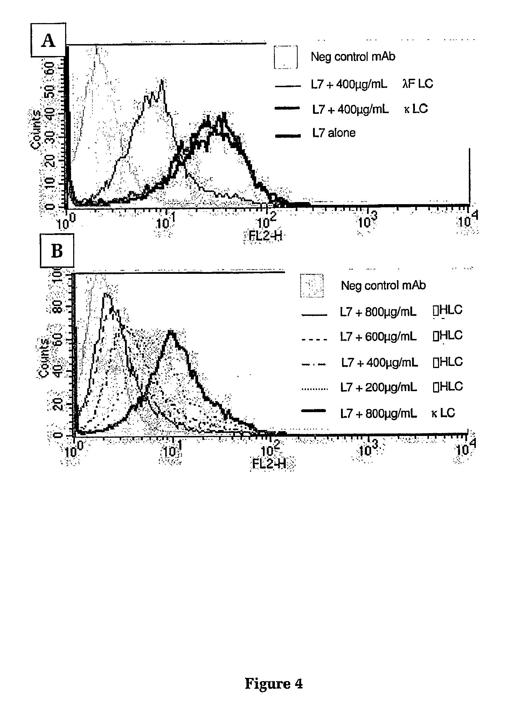

**Figure 4****.** Pre-incubation with free λLC inhibits the binding of mAb L7 to LP-1 cells. MAb L7 (100µg/mL) was pre-incubated with free lambda (λF LC, A or λH LC, B) or kappa (κLC) light chain at concentrations from 200-800µg/mL for 30 minutes at 37°C. Cells were then incubated with the inhibited L7 for 30 minutes on ice, washed twice and incubated with PE-labelled goat-anti-mouse F(ab')₂. After two washes cells were analysed by flow cytometry. The solid grey histogram represents cells which were incubated with an-anti κLC mAb (negative control) followed by the PE-labelled goat-anti-mouse F(ab')₂.

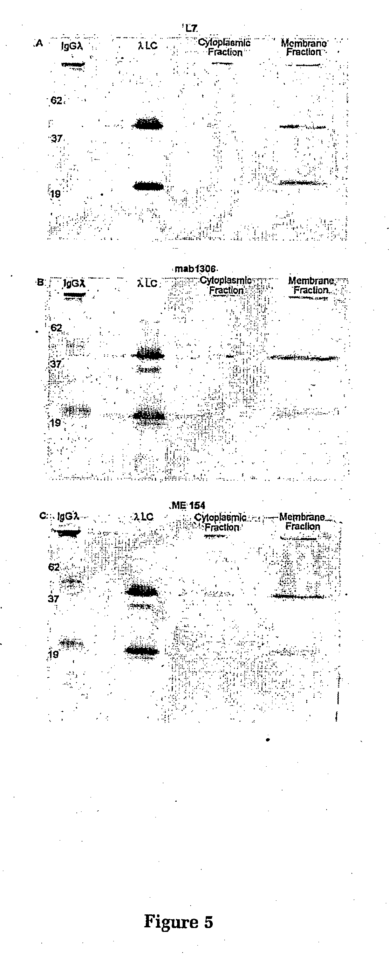

**Figure 5****.** Identifcation of a free λ LC antigen on the surface of LP-1 λmyeloma cells by Western blot. LP-1 cells were lysed in 40mM Tris and the cytoplasmic and membrane-bound protein fractions were isolated under non-reducing conditions. Fractions were seperated by 12% SDS-PAGE, transferred to nitrocellulose membrane. After BSA blocking of the membrane, λ light chains were detected with anti-λ mAbs L7 (A), mab1306 (B) or ME 154 (C). Numbers on the left edge of the figures are approximate molecular weights (kDa).

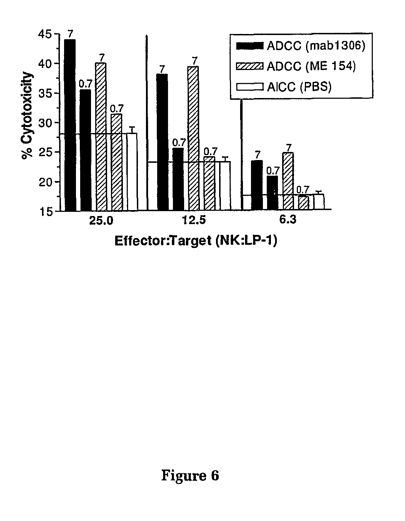

**Figure 6****.** MAbs binding to lambda light chains induce antibody-dependent cell-mediated cytotoxicity (ADCC) against LP-1 myeloma cells. PKH-26 labelled LP-1 myeloma cells were incubated with 7nM (1µg/mL) or 0.7nM mab1306 (dark bars) or ME 154 (hatched bars) in the presence of IL-2 stimulated murine NK cells. After 16 hours dead LP-1 cells were detected by TO-PRO-Iodide 3 fluorescence by flow cytometer, and the percentage of dead LP-1 cells was calculated. To control for Antibody Independent Cell-mediated Cytotoxicity (AICC), LP-1 cells were also incubated in PBS (clear bars) without antibody in the presence of NK effector cells (Error bars show triplicate SEM).

### Detailed Description of the Invention

### General Techniques

Unless specifically defined otherwise, all technical and scientific terms used herein shall be taken to have the same meaning as commonly understood by one of ordinary skill in the art (e.g., in cell culture, molecular genetics, immunology, immunohistochemistry, protein chemistry, and biochemistry).

Unless otherwise indicated, the recombinant protein, cell culture, and immunological techniques utilized in the present invention are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

### anti-LMA antibodies

The present inventors have now shown, for the first time, that free lambda light chain (LMA) is expressed on the surface of myeloma cells. It is envisaged that antibodies directed against LMA will be capable of killing lambda-type myeloma cells through mechanisms such as ADCC, complement dependent lysis and apoptosis and will therefore be effective therapeutic agents against lambda-type myeloma cells. In addition, antibodies directed against LMA can be used to deliver cytotoxins directly to malignant cells.

It will be known to those skilled in the art that a lambda-type immunoglobulin consists of four polypeptide chains, whereby two of these are heavy chains (each about 50-70 kD) and two are lambda light chains (each about 26 kD. The N-terminus of each chain defines a variable region of about 100 to 110 or more amino acids primarily responsible for antigen recognition. The terms variable light chain (V_{L}) and variable heavy chain (V_{H}) refer to these light and heavy chains respectively. The assembly of the gene encoding lambda light chains involves rearrangement of the V, J and C gene segments at the lambda light chain locus (see Chapter 5, "Organization and Expression of Immunoglobulin Genes" in Kuby Immunology, 4th ed, Goldsby et al. (Freeman, 2000)).

Accordingly, when used herein the term "LMA" encompasses any free lambda light chain equivalent to a light chain derived from a lambda-type immunoglobulin. The term therefore encompasses a range of lambda light chain polypeptides that can differ in their variable region sequences.

In a preferred embodiment of the invention, the LMA is approximately 26kD in the monomeric form and approximately 52kD in the dimeric form.

The LMA ligand is preferably capable of binding to LMA when the LMA is membrane bound or in an isolated state (e.g. free of a membrane). LMA ligands are anti-LMA antibodies.

The LMA ligand binds specifically to LMA. The phrase "bind specifically," means that under particular conditions, the LMA ligand binds LMA and does not bind to a significant amount to other proteins or carbohydrates. Specific binding to LMA under such conditions may require an antibody that is selected for its specificity. A variety of immunoassay formats may be used to select antibodies specifically immunoreactive with LMA. For example, solid-phase ELISA immunoassays are routinely used to select antibodies specifically immunoreactive with a protein or carbohydrate. See Harlow and Lane (1988) Antibodies, a Laboratory Manual, Cold Spring Harbor Publications, New York, for a description of immunoassay formats and conditions that can be used to determine specific immunoreactivity.

Anti-LMA antibodies will be known to those skilled in the art. For example, antibodies directed against LMA have been used to detect free lambda light chains in serum or urine in tests for diagnosing multiple myeloma (Bradwell et at., (2001) Clin. Chem. 47:673-680). LMA has not been used to date, however, as a target for the treatment of multiple myeloma or for the localization of myeloma cells in a patient.

Examples of suitable anti-LMA antibodies include RDI-TRK1L7-3D1 (RDI; Flanders, NJ, USA), CBL317 (Cymbus Biotechnology Ltd, UK), 2G7 (Nakano and Nagata (2003) J Immunol Methods 275; 9-17), L7 or ME-154 (AbCam Ltd (Cambridge, UK) or mAb1306 (Chemicon International, Australia).

Antibodies may exist as intact immunoglobulins, or as modifications in a variety of forms including, for example, domain antibodies including either the VH or VL domain, a dimer of the heavy chain variable region (VHH, as described for a camelid), a dimer of the light chain variable region (VLL), Fv fragments containing only the light and heavy chain variable regions, or Fd fragments containing the heavy chain variable region and the CHI domain. A scFv consisting of the variable regions of the heavy and light chains linked together to form a single-chain antibody (Bird et al., Science, 242: 424-426 (1988); Huston et al., Proc. Natl, Acad. Sci. USA, 85: 5879-5883 (1988)) and oligomers of scFvs such as diabodies and triabodies are also encompassed by the term "antibody". Also encompassed are fragments of antibodies such as Fab, (Fab')2 and FabFc₂ fragments which contain the variable regions and parts of the constant regions. CDR-grafted antibody fragments and oligomers of antibody fragments are also encompassed. The heavy and light chain components of an Fv may be derived from the same antibody or different antibodies thereby producing a chimeric Fv region. The antibody may be of animal (especially mouse or rat) or human origin or may be chimeric (Monason et al., Proc. Natl. Acad. Sci. USA, 81, 6851-6855 (1984)) or humanized (Jones et al., Nature, 321, 522-525 (1986), and published UK patent application #8707252). As used herein the term "antibody" includes these various forms. Using the guidelines provided herein and those methods well known to those skilled in the art which are described in the references cited above and in such publications as Harlow & Lane, Antibodies: a Laboratory Manual, Cold Spring Harbor Laboratory, (1988) the antibodies of the present invention can be readily made.

The LMA-binding antibodies may be Fv regions comprising a variable light (V_{L}) and a variable heavy (V_{H}) chain. The light and heavy chains may be joined directly or through a linker. As used herein a linker refers to a molecule that is covalently linked to the light and heavy chain and provides enough spacing and flexibility between the two chains such that they are able to achieve a conformation in which they are capable of specifically binding the epitope to which they are directed. Protein linkers are particularly preferred as they may be expressed as an intrinsic component of the Ig portion of the fusion polypeptide.

Another preferred embodiment of the invention is a recombinantly produced single chain scFv antibody, preferably a humanized scFv.

### Preparation of Genes Encoding Antibodies or Fragments Thereof

Genes encoding antibodies, both light and heavy chain genes or portions thereof, e.g., single chain Fv regions, may be cloned from a hybridoma cell line. They may all be cloned using the same general strategy. Typically, for example, poly(A)⁺mRNA extracted from the hybridoma cells is reverse transcribed using random hexamers as primers. For Fv regions, the V_{H} and V_{L} domains are amplified separately by two polymerase chain reactions (PCR). Heavy chain sequences may be amplified using 5' end primers which are designed according to the amino-terminal protein sequences of the anti-LMA heavy chains respectively and 3' end primers according to consensus immunoglobulin constant region sequences (Kabat et al., Sequences of Proteins of Immunological Interest. 5th edition. U.S. Department of Health and Human Services, Public Health Service, National Institutes of Health, Bethesda, Md. (1991)). Light chain Fv regions are amplified using 5' end primers designed according to the amino-terminal protein sequences of anti-LMA light chains and in combination with the primer C-kappa. One of skill in the art would recognize that many suitable primers may be employed to obtain Fv regions.

The PCR products are subcloned into a suitable cloning vector. Clones containing the correct size insert by DNA restriction are identified. The nucleotide sequence of the heavy or light chain coding regions may then be determined from double stranded plasmid DNA using sequencing primers adjacent to the cloning site. Commercially available kits (e.g., the Sequenase.TM. kit, United States Biochemical Corp., Cleveland, Ohio, USA) may be used to facilitate sequencing the DNA.

Thus, DNA encoding the Fv regions may be prepared by any suitable method, including, for example, amplification techniques such as ligase chain reaction (LCR) (see Wu and Wallace, Genomics, 4: 560 (1989), Landegren, et al., Science, 241: 1077 (1988) and Barringer, et al., Gene, 89: 117 (1990)), transcription amplification (see Kwoh, et al., Proc. Natl. Acad. Sci. USA, 86: 1173 (1989)), and self-sustained sequence replication (see Guatelli, et al., Proc. Natl. Acad. Sci. USA, 87: 874 (1990)), cloning and restriction of appropriate sequences or direct chemical synthesis by methods such as the phosphotriester method of Narang et al., Meth. Enzymol. 68: 90-99 (1979); the phosphodiester method of Brown et al., Meth Enzymol. 68: 109-151 (1979); the diethylphosphoramidite method of Beaucage et al., Tetra. Lett., 22: 1859-1862 (1981); and the solid support method of U.S. Pat. No. 4,458,066.

Chemical synthesis produces a single stranded oligonucleotide. This may be converted into double stranded DNA by hybridization with a complementary sequence, or by polymerization with a DNA polymerase using the single strand as a template. While it is possible to chemically synthesize an entire single chain Fv region, it is preferable to synthesize a number of shorter sequences (about 100 to 150 bases) that are later ligated together.

Alternatively, sub-sequences may be cloned and the appropriate subsequences cleaved using appropriate restriction enzymes. The fragments may then be ligated to produce the desired DNA sequence.

Once the Fv variable light and heavy chain DNA is obtained, the sequences may be ligated together, either directly or through a DNA sequence encoding a peptide linker, using techniques well known to those of skill in the art. In one embodiment, heavy and light chain regions are connected by a flexible peptide linker (e.g., (Gly₄Ser)₃) which starts at the carboxyl end of the heavy chain Fv domain and ends at the amino terminus of the light chain Fv domain. The entire sequence encodes the Fv domain in the form of a single-chain antigen binding protein.

### Cytotoxic moieties

Suitable cytotoxic moieties for use in the present invention includes, but is not limited to, agents such as bacterial or plant toxins, drugs, e.g., cyclophosphamide (CTX; cytoxan), chlorambucil (CHL; leukeran), cisplatin (CisP; CDDP; platinol), busulfan (myleran), melphalan, carmustine (BCNU), streptozotocin, triethylenemelamine (TEM), mitomycin C, and other alkylating agents; methotrexate (MTX), etoposide (VP-16; vepesid), 6-mercaptopurine (6MP), 6-thioguanine (6TG), cytarabine (Ara-C), 5-fluorouracil (5FU), dacarbazine (DTIC), 2-chlorodeoxyadenosine (2-CdA), and other antimetabolites; antibiotics including actinomycin D, doxorubicin (DXR; adriamycin), daunorubicin (daunomycin), bleomycin, mithramycin as well as other antibiotics; alkaloids such as vincristin (VCR), vinblastine, and the like; as well as other anticancer agents including the cytostatic agents glucocorticoids such as dexamethasone (DEX; decadron) and corticosteroids such as prednisone, nucleotide enzyme inhibitors such as hydroxyurea, and the like.

Those skilled in the art will realize that there are numerous other radioisotopes and chemocytotoxic agents that can be coupled to LMA ligands by well known techniques, and delivered to specifically destroy tumor tissue. See, e.g., U.S. Pat. No. 4,542,225 to Blattler et al. Examples of photo-activated toxins include dihydropyridine-and omega-conotoxin (Schmidt et al., J Biol. Chem., 1991, 266(27):18025-33). Examples of imaging and cytotoxic reagents that can be used include ¹²⁵I, ¹³¹I, ¹¹¹In, ¹²³I, ⁹⁹mTc, ³²P, ³H, and ¹⁴C; fluorescent labels such as fluorescein and rhodamine, and chemiluminescers such as luciferin. The antibody can be labeled with such reagents using techniques known in the art. For example, see Wenzel and Meares, Radioimmunoimaging and Radioimmunotherapy, Elsevier, N.Y. (1983) for techniques relating to the radiolabeling of antibodies (see also, Colcer et al., "Use of Monoclonal Antibodies As Radiopharmaceuticals For The Localization Of Human Carcinoma Xenografts In Nude Mice", Methods Enzymol., 121:802-16, 1986: "Order, Analysis, Results and Future Prospective of the Therapeutic Use of Radiolabeled Antibody in Cancer Therapy", in Monoclonal Antibodies for Cancer Detection and Therapy, Baldwin et al. (eds), pp. 303-16 (Academic Press 1985)).

In one example, the linker-chelator tiuexutan is conjugated to a LMA ligand, by a stable thiourea covalent bond to provide a high-affinity chelation site for Indium-111 or Yttrium-90.

When a DNA molecule encoding a cytotoxic agent is present in a therapeutic composition of the invention, the DNA preferably encodes a polypeptide that is a bacterial or plant toxin. These polypeptides include, but are not limited to, polypeptides such as native or modified Pseudomonas exotoxin (PE), diphtheria toxin (DT), ricin, abrin, gelonin, momordin II, bacterial RIPs such as shiga and shiga-like toxin a-chains, luffin [see Islam et al., Agricultural Biological Chem., 54(5):1343-1345 (1990)], atrichosanthin [see Chow et al., J. Biol. Chem., 265:8670-8674 (1990))], momordin I [see Ho et al., BBA, 1088:311-314 (1991)], Mirabilis anti-viral protein [see Habuka et al., J. Biol. Chem., 264(12):6629-6637 (1989)], pokeweed antiviral protein [see Kung et al., Agric. Biol. Chem., 54(12):3301-3318 (1990)], byodin 2 (U.S. Pat. No. 5,597,569), gaporin [see Benatti et al., Eur. J. Biochem., 183:465-470 (1989)], as well as genetically engineered variants thereof. Native PE and DT are highly toxic compounds that typically bring about death through liver toxicity. Preferably, PE and DT are modified into a form that removes the native targeting component of the toxin, e.g., domain Ia of PE and the B chain of DT. One of skill in the art will appreciate that the invention is not limited to a particular cytotoxic agent.

The term "Pseudomonas exotoxin" (PE) as used herein refers to a full-length native (naturally occurring) PE or a PE that has been modified. Such modifications may include, but are not limited to, elimination of domain Ia, various amino acid deletions in domains II and III, single amino acid substitutions (e.g., replacing Lys with Gln at positions 590 and 606), and the addition of one or more sequences at the carboxyl terminus. See Siegall et al., J. Biol. Chem., 264: 14256-14261 (1989). Thus, for example, PE38 refers to a truncated Pseudomonas exotoxin composed of amino acids 253-364 and 381-613. The native C-terminus of PE, REDLK (residues 609-613), may be replaced with the sequence KDEL, REDL, and Lys-590 and Lys-606 may be each mutated to Gln.

The term "Diphtheria toxin" (DT) as used herein refers to full length native DT or to a DT that has been modified. Modifications typically include removal of the targeting domain in the B chain and, more specifically, involve truncations of the carboxyl region of the B chain.

### Preparation of Antibody Fusion Polypeptides

Once a DNA sequence has been identified that encodes an LMA binding moiety (eg. an anti-LMA antibody fragment) fusion polypeptides comprising that region may be prepared by methods known to one of skill in the art. For example, a gene encoding an Fv region is fused to a gene encoding a cytotoxic moiety, preferably a moiety which is a polypeptide. Optionally, the Fv gene is linked to a segment encoding a peptide connector. The peptide connector may be present simply to provide space between the LMA binding moiety and the cytotoxic moiety or to facilitate mobility between these regions to enable them to each attain their optimum conformation. The DNA sequence comprising the connector may also provide sequences (such as primer sites or restriction sites) to facilitate cloning or may preserve the reading frame between the sequence encoding the binding moiety and the sequence encoding the cytotoxic moiety. The design of such connector peptides is well known to those of skill in the art.

Generally producing fusion polypeptides involves separately preparing the Fv light and heavy chains and DNA encoding any other protein to which they are fused and recombining the DNA sequences in a plasmid or other vector to form a construct encoding the particular desired fusion polypeptide. However, a simpler approach involves inserting the DNA encoding the particular Fv region into a construct already encoding the desired second polypeptide. The DNA sequence encoding the Fv region is inserted into the construct using techniques well known to those of skill in the art.

One embodiment of the invention is a fusion polypeptide comprising a recombinantly produced antibody comprising a V_{H} and C_{H}, or a portion thereof, joined to a DNA binding polypeptide. The fusion polypeptide and an antibody comprising V_{L} and C_{L}, or a portion thereof, together form a recombinant antibody useful to direct preselected DNA molecules, either linear or circular, to a cell or tissue bearing the preselected target molecule.

Another preferred embodiment of the invention is a recombinantly produced single chain scFv antibody, preferably a humanized scFv. In particular, this invention provides for recombinant single chain antibodies that are joined to a DNA binding polypeptide and, because of their ability to specifically bind to DNA, these antibodies are useful as targeting moieties which serve to direct DNA which is bound to DNA binding polypeptide to a cell or tissue bearing LMA.

The recombinant single chain antibodies of the present invention may be fused to, or otherwise bound to the cytotoxin or other molecule having a specified activity by any method known and available to those in the art. The two components may be chemically bonded together by any of a variety of well-known chemical procedures. For example, the linkage may be by way of heterobifunctional cross-linkers, e.g., SPDP, carbodiimide, glutaraldehyde, or the like. Production of various immunotoxins, as well as chemical conjugation methods, are well-known within the art and can be found, for example in "Monoclonal Antibody-Toxin Conjugates: Aiming the Magic Bullet," Thorpe et al., Monoclonal Antibodies in Clinical Medicine, Academic Press, pp. 168-190 (1982); Waldmann, Science, 252: 1657 (1991); Vitetta et al., 1987, Science, 238:1098; Pastan et al., 1986; Cell, 47:641; and Thorpe et al., 1987, Cancer Res., 47:5924. These methods generally conjugate the cytotoxin and the antibody by means of cross-linking agents that introduce a disulfide bond between the two polypeptides. Immunotoxins which have been prepared with nonreducible linkages have been shown to be consistently less cytotoxic than similar toxins cross-linked by disulfide bonds.

Other preferred reagents are 2-iminothiolane hydrochloride (2IT), sodium S-4-succinimidyloxycarbonyl-.alpha.-methyl benzyl thiosulfate (SMBT) and 2IT or succinimidyloxy carbonyl-α-methyl-α(2-pyrid- yldithio)-toluene and 2IT. Each group of reagents introduces a disulfide bond between the cytotoacin and the antibody which is reducible, but the bond is also resistant to breakdown providing stability of the conjugate *in vitro* and *in vivo.* Upon internalization into lysosomes or endosomes by the target cell, the bond is reduced. For example, to use the recombinant PE molecules with an antibody, a form of the PE molecule with cysteine at amino acid position 287 is preferred to couple the toxin to the antibody or other ligand through the thiol moiety of cysteine.

In one embodiment, the LMA binding moiety may also be fused to a cytotoxin by recombinant means such as through the use of recombinant DNA techniques to produce a nucleic acid which encodes both the antibody and the cytotoxin and expressing the recombinant DNA sequence in a host cell, such as a eukaryotic, e.g., mammalian such as CHO or COS cells, or prokaryotic, e.g., *E. coli,* host. The DNA encoding the fusion polypeptide may be cloned in cDNA or in genomic form by any cloning procedure known to those skilled in the art. See for example Sambrook et al., Molecular Cloning: a Laboratory Manual, Cold Spring Harbor Laboratory, (1989).

One of skill in the art would recognize that after chemical synthesis, biological expression, or purification, a fusion polypeptide may possess a conformation substantially different than the native antibody. In this case, it may be necessary to denature and reduce the polypeptide and then to cause the polypeptide to re-fold into the preferred conformation. Methods of reducing and denaturing the polypeptide and inducing re-folding are well known to those of skill in the art. (See, Debinski et al., J. Biol. Chem, 268: 14065-14070 (1993); Kreitman and Pastan, Bioconjug. Chem., 4: 581-585 (1993); and Buchner, et al., Anal. Biochem., 205: 263-270 (1992)) Debinski *et al.,* for example, describe the denaturation and reduction of inclusion body proteins in guanidine-DTE. The polypeptide is then refolded in a redox buffer containing oxidized glutathione and L-arginine.

One of skill would recognize that modifications can be made to the fusion polypeptides without diminishing their biological activity. Some modifications may be made to facilitate the cloning, expression, or incorporation of the antibody portion of the fusion polypeptide into the fusion polypeptide. Such modifications are well known to those of skill in the art and include, for example, a methionine added at the amino terminus to provide an initiation site, a His-Tag added at either end of the polypeptide to facilitate purification or additional amino acids placed on either terminus to create conveniently located restriction sites or termination codons.

One of skill will recognize that other modifications may be made. Thus, for example, amino acid substitutions may be made that increase specificity or binding affinity of the fusion polypeptide. Alternatively, non-essential regions of the molecule may be shortened or eliminated entirely. Thus, where there are regions of the molecule that are not themselves involved in the activity of the molecule, they may be eliminated or replaced with shorter segments that serve to maintain the correct spatial relationships between the active components of the molecule. Alternatively more flexible segments may be placed in interdomain regions which then can facilitate folding or production of the molecule (Brinkmann, et al., Proc. Natl. Acad. Sci. USA, 89: 3075-3079 (1992).

### Monoclonal antibodies

Monoclonal antibodies directed against LMA epitopes can be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against LMA epitopes can be screened for various properties; i.e. for isotype and epitope affinity.

Mouse-derived monoclonal antibodies can be used for both direct *in vivo* and extracorporeal immunotherapy. However, it has been observed that when mouse-derived monoclonal antibodies are used in humans as therapeutic agents, the patient produces human anti-mouse antibodies. Thus, mouse-derived monoclonal antibodies are not preferred for therapy, especially for long term use. With established genetic engineering techniques it is possible, however, to create chimeric or humanized antibodies that have animal-derived and human-derived portions. The animal can be a mouse or another rodent such as a rat.

If the variable region of the chimeric antibody is mouse-derived while the constant region is human-derived, the chimeric antibody will generally be less immunogenic than a "pure" mouse-derived monoclonal antibody. These chimeric antibodies would likely be more suited for therapeutic use, should it turn out that "pure" mouse-derived antibodies are unsuitable.

### Chimeric Antibodies

Methodologies for generating chimeric antibodies are available to those in the art. For example, the light and heavy chains can be expressed separately, using, for example, immunoglobulin light chain and immunoglobulin heavy chains in separate plasmids. These can then be purified and assembled *in vitro* into complete antibodies; methodologies for accomplishing such assembly have been described. See, for example, Scharff, M., Harvey Lectures 69:125 (1974). See also Oi et al., Bio Techniques 4(4):214-221 (1986); and Sun et al. Hybridoma 5 (1986) Suppl 1:517-20. Such a DNA construct may comprise DNA encoding functionally rearranged genes for the variable region of a light or heavy chain of an anti-LMA antibody linked to DNA encoding a human constant region. Lymphoid cells such as myelomas or hybridomas transfected with the DNA constructs for light and heavy chain can express and assemble the antibody chains.

*In vitro* reaction parameters for the formation of IgG antibodies from reduced isolated light and heavy chains have also been described. See, for example, Beychok, S., Cells of Immunoglobulin Synthesis, Academic Press, New York, p. 69,1979. Co-expression of light and heavy chains in the same cells to achieve intracellular association and linkage of heavy and light chains into complete H2L2 IgG antibodies is also possible. Such co-expression can be accomplished using either the same or different plasmids in the same host cell.

### Humanised antibodies

In another preferred embodiment of the present invention the anti-LMA antibody is humanised, that is, an antibody produced by molecular modeling techniques wherein the human content of the antibody is maximised while causing little or no loss of binding affinity attributable to the variable region of the murine antibody.

The methods described below are applicable to the humanisation of anti-LMA antibodies.

There are several factors to consider in deciding which human antibody sequence to use during the humanisation. The humanisation of light and heavy chains are considered independently of one another, but the reasoning is basically similar for each.

This selection process is based on the following rationale: A given antibody's antigen specificity and affinity is primarily determined by the amino acid sequence of the variable region CDRs. Variable domain framework residues have little or no direct contribution. The primary function of the framework regions is to hold the CDRs in their proper spatial orientation to recognize antigen. Thus the substitution of rodent CDRs into a human variable domain framework is most likely to result in retention of their correct spatial orientation if the human variable domain framework is highly homologous to the rodent variable domain from which they originated. A human variable domain should preferably be chosen therefore that is highly homologous to the rodent variable domain(s). A suitable human antibody variable domain sequence can be selected as follows;

Step 1. Using a computer program, search all available protein (and DNA) databases for those human antibody variable domain sequences that are most homologous to the rodent antibody variable domains. The output of a suitable program is a list of sequences most homologous to the rodent antibody, the percent homology to each sequence, and an alignment of each sequence to the rodent sequence. This is done independently for both the heavy and light chain variable domain sequences. The above analyses are more easily accomplished if only human immunoglobulin sequences are included.

Step 2. List the human antibody variable domain sequences and compare for homology. Primarily the comparison is performed on length of CDRs, except CDR3 of the heavy chain which is quite variable. Human heavy chains and Kappa and Lambda light chains are divided into subgroups; Heavy chain 3 subgroups, Kappa chain 4 subgroups, Lambda chain 6 subgroups. The CDR sizes within each subgroup are similar but vary between subgroups. It is usually possible to match a rodent antibody CDR to one of the human subgroups as a first approximation of homology. Antibodies bearing CDRs of similar length are then compared for amino acid sequence homology, especially within the CDRs, but also in the surrounding framework regions. The human variable domain which is most homologous is chosen as the framework for humanisation.

### The Actual Humanising Methodologies/Techniques

An antibody may be humanised by grafting the desired CDRs onto a human framework according to EP-A-0239400. A DNA sequence encoding the desired reshaped antibody can therefore be made beginning with the human DNA whose CDRs it is wished to reshape. The rodent variable domain amino acid sequence containing the desired CDRs is compared to that of the chosen human antibody variable domain sequence. The residues in the human variable domain are marked that need to be changed to the corresponding residue in the rodent to make the human variable region incorporate the rodent CDRs. There may also be residues that need substituting in, adding to or deleting from the human sequence.

Oligonucleotides are synthesized that can be used to mutagenize the human variable domain framework to contain the desired residues. Those oligonucleotides can be of any convenient size. One is normally only limited in length by the capabilities of the particular synthesizer one has available. The method of oligonucleotide-directed in vitro mutagenesis is well known.

Alternatively, humanisation may be achieved using the recombinant polymerase chain reaction (PCR) methodology of WO 92/07075. Using this methodology, a CDR may be spliced between the framework regions of a human antibody.

In general, the technique of WO 92/07075 can be performed using a template comprising two human framework regions, AB and CD, and between them, the CDR which is to be replaced by a donor CDR. Primers A and B are used to amplify the framework region AB, and primers C and D used to amplify the framework region CD. However, the primers B and C each also contain, at their 5' ends, an additional sequence corresponding to all or at least part of the donor CDR sequence. Primers B and C overlap by a length sufficient to permit annealing of their 5' ends to each other under conditions which allow a PCR to be performed. Thus, the amplified regions AB and CD may undergo gene splicing by overlap extension to produce the humanised product in a single reaction.

### The Transfection and Expression of the Reshaped Antibody

Following the mutagenesis reactions to reshape the antibody, the mutagenised DNAs can be linked to an appropriate DNA encoding a light or heavy chain constant region, cloned into an expression vector, and transfected into host cells, preferably mammalian cells. These steps can be carried out in routine fashion. A reshaped antibody may therefore be prepared by a process comprising:
(a) preparing a first replicable expression vector including a suitable promoter operably linked to a DNA sequence which encodes at least a variable domain of an Ig heavy or light chain, the variable domain comprising framework regions from a human antibody and the CDRs required for the humanised antibody of the invention;
(b) preparing a second replicable expression vector including a suitable promoter operably linked to a DNA sequence which encodes at least the variable domain of a complementary Ig light or heavy chain respectively;
(c) transforming a cell line with the first or both prepared vectors; and
(d) culturing said transformed cell line to produce said altered antibody.

Preferably the DNA sequence in step (a) encodes both the variable domain and each constant domain of the human antibody chain. The humanised antibody can be prepared using any suitable recombinant expression system. The cell line which is transformed to produce the altered antibody may be a Chinese Hamster Ovary (CHO) cell line or an immortalised mammalian cell line, which is advantageously of lymphoid origin, such as a myeloma, hybridoma, trioma or quadroma cell line. The cell line may also comprise a normal lymphoid cell, such as a B-cell, which has been immortalised by transformation with a virus, such as the Epstein-Barr virus. Most preferably, the immortalised cell line is a myeloma cell line or a derivative thereof.

The CHO cells used for expression of the antibodies according to the invention may be dihydrofolate reductase (dhfr) deficient and so dependent on thymidine and hypoxanthine for growth (Urlaub et al., Proc. Natl. Acac. Sci. U.S.A., 77 4216-4220 (1980)). The parental dhfr⁻ CHO cell line is transfected with the DNA encloding the antibody and dhfr gene which enables selection of CHO cell transformants of dhfr positive phenotype. Selection is carried out by culturing the colonies on media devoid of thymidine and hypoxanthine, the absence of which prevents untransformed cells from growing and transformed cells from resalvaging the folate pathway and thus bypassing the selection system. These transformants usually express low levels of the DNA of interest by virtue of co-integration of transfected DNA of interest and DNA encoding dhfr. The expression levels of the DNA encoding the antibody may be increased by amplification using methotrexate (MTX). This drug is a direct inhibitor of the enzyme dhfr and allows isolation of resistant colonies which amplify their dhfr gene copy number sufficiently to survive under these conditions. Since the DNA sequences encoding dhfr and the antibody are closely linked in the original transformants, there is usually concomitant amplification, and therefore increased expression of the desired antibody.

Another preferred expression system for use with CHO or myeloma cells is the glutamine synthetase (GS) amplification system described in WO 87/04462. This system involves the transfection of a cell with DNA encoding the enzyme GS and with DNA encoding the desired antibody. Cells are then selected which grow in glutamine free medium and can thus be assumed to have integrated the DNA encoding GS. These selected clones are then subjected to inhibition of the enzyme GS using methionine sulphoximine (Msx). The cells, in order to survive, will amplify the DNA encoding GS with concomitant amplification of the DNA encoding the antibody.

Although the cell line used to produce the humanised antibody is preferably a mammalian cell line, any other suitable cell line, such as a bacterial cell line or a yeast cell line, may alternatively be used. In particular, it is envisaged that E. coli--derived bacterial strains could be used. The antibody obtained is checked for functionality. If functionality is lost, it is necessary to return to step (2) and alter the framework of the antibody.

Once expressed, the whole antibodies, their dimers, individual light and heavy chains, or other immunoglobulin forms of the present invention can be recovered and purified according to standard procedures of the art, including ammonium sulfate precipitation, affinity columns, column chromatography, gel electrophoresis and the like (See, generally, Scopes, R., Protein Purification, Springer-Verlag, N.Y. (1982)). Substantially pure immunoglobulins of at least about 90 to 95% homogeneity are preferred, and 98 to 99% or more homogeneity most preferred, for pharmaceutical uses. Once purified, partially or to homogeneity as desired, a humanised antibody may then be used therapeutically or in developing and performing assay procedures, immunofluorescent stainings, and the like (See, generally, Immunological Methods, Vols. I and II, Lefkovits and Pernis, eds., Academic Press, New York, N.Y. (1979 and 1981)).

Studies carried out by Greenwood and Clark ((1993) Eur. J. Immunol. 23:1098-1104) have demonstrated that recognition of the Fc region of an antibody by human effector cells can be optimised by engineering the constant region of the immunoglobulin molecule. This could be achieved by fusing the variable region genes of the antibody, with the desired specificity, to human constant region genes encoding immunoglobulin isotypes that have demonstrated effective ADCC in human subjects, for example the IgG1 and IgG3 isotypes (Greenwood and Clark (1993) Protein Engineering of Antibody Molecules for Prophylactic and Therapeutic Applications in Man. Edited by Mike Clark, published by Academic Titles. Section II 85-113). The resulting chimeric or humanised antibodies to LMA should be particularly effective in inducing ADCC.

Antibodies with fully human variable regions against LMA can also be prepared by administering the antigen to a transgenic animal which has been modified to produce such antibodies in response to antigenic challenge, but whose endogenous loci have been disabled. Various subsequent manipulations can be performed to obtain either antibodies per se or analogs thereof (see, for example, US Patent No. 6,075,181).

In one aspect the use of the present invention utilizes the antibodies or binding fragments without modification, relying on the binding of the antibodies or fragments to the surface LMAs of the myeloma cells in situ to stimulate an immune attack thereon. For example, a chimeric antibody, wherein the antigen-binding site is joined to human Fc region, e.g., IgG1, may be used to promote antibody-dependent mediated cytotoxicity or complement-mediated cytotoxicity.

In another aspect of the invention, the use may be carried out using LMA binding moieties to which a cytotoxic agent or biological modifier is bound. Binding of the resulting conjugate to the tumor cells inhibits the growth of or kills the cells.

Anti-idiotypic monoclonal antibodies to the antibodies of the invention may also be used therapeutically in active tumor immunization and tumor therapy (see, e.g., Hellstrom et al., "Anti Idiotypes" in Covalently Modified Antigens and Antibodies in Diagnosis and Therapy, supra at pp. 35-41).

In the area of multiple myeloma, the antibodies or antibody fragments of the present invention have further utility in the preparation of cellular samples from which myeloma cells have been removed. This use is particularly important in autologous bone marrow transplants, wherein a sample of bone marrow is harvested from a cancer patient prior to the patient's undergoing high-dose chemotherapy. The goal of the high dose chemotherapy is to destroy the cancer cells, which also results in the depletion of bone marrow cells. Following such treatment, the harvested bone marrow cells are reintroduced into the patient.

In myeloma and related diseases, the harvested bone marrow is contaminated with myeloma cells; thus, reintroduction of untreated bone marrow will simply reintroduce the disease. Previous methods to prevent reintroduction of cancer cells have included treatment of the bone marrow sample with chemotherapeutic agents and other antineoplastic agents in vitro. Other methods include purging the sample of cancer cells.

In a further practice of the present invention, the monoclonal antibodies and fragments described herein may be used to remove myeloma cells from a patient's bone marrow sample before reintroduction into the patient. In one non-limiting example, the monoclonal antibodies, or binding fragments, are attached to a matrix, such as beads. This may be accomplished by any of several well-known methods for preparing an affinity matrix comprising antibodies or their binding fragments. The bone marrow sample is then exposed to the matrix, such as by passage of the cells over a column containing the matrix, under conditions to promote the binding of the myeloma cells in the sample through antigen/antibody interactions with the antibodies or binding fragments attached to the matrix. The myeloma cells in the sample adhere to the matrix; while the column effluent, i.e., the non-adherent cellular population, is depleted of myeloma cells. The effectiveness of the procedure may be monitored by examining the cells for residual myeloma cells, such as by using a detectably-labeled antibody as described below. The procedure may be repeated or modified to increase effectiveness.

This purging procedure (see, e.g., Ramsay et al., J. Clin. Immunol., 8(2):81-88, 1988) may be performed together with other methods for removing or killing cancer cells, including, but not limited to, exposing the purified bone marrow cells to chemotherapeutic agents. Such chemotherapeutic agents include the use of the antibodies or antibody binding fragments of the present invention conjugated to a cytotoxic agent, as those described above for in vivo therapeutic treatment. Accordingly, conjugates of the antibodies or antibody fragments of the present invention with cytotoxic agents may be used for the ex vivo killing of tumor cells in a cellular sample. The methods may additionally include exposing the cells to cytokines (e.g., GM-CSF, IL-6), cytokine receptors (e.g., IL-6-receptor), mitogens (e.g., poke weed mitogen (PWM)), or adhesion molecules (e.g., CD40 ligand) in order to stimulate the myeloma cells to rapidly differentiate and thereby upregulate expression of cancer-specific antigens on their cell surface. These treatment modalities are intended to render the myeloma cells vulnerable to the *in vitro-* mediated cytotoxicity achieved by incubation with the monoclonal antibody, or fragments thereof, according to the present invention.

In another aspect of the therapeutic methods of the present invention, the antibodies, or binding fragments thereof, conjugated with cytotoxic agents, such as chemotherapeutic agents, a photo-activatable toxin, or a radionuclide, may be used *in vitro* or *ex vivo* to inhibit or kill myeloma cells from a bone marrow sample, in the absence of the purging technique described above. The treatment of a sample with the cytotoxic antibodies, or antibody fragments, may be combined with other methods to kill cancer cells to increase the effectiveness of a bone marrow transplant, particularly an autologous bone marrow transplant, by removing cells from the tissue to be transplanted. These methods may include additionally exposing the cells to cytokines, etc. Thus, a method is described herein for removing myeloma cells from an isolated cellular sample comprising the steps of exposing the cellular sample to a solid matrix on which a monoclonal antibody, or antibody binding fragment as described herein, is bound under conditions in which the myeloma cells adhere to the monoclonal antibody, or binding fragment thereof, and isolating a cellular fraction of the cellular sample which does not bind to the matrix. By way of non-limiting example, bone marrow cells are used, particularly for a transplant, and preferably, an autologous bone marrow transplant.

As will be appreciated by those skilled in the art, some myeloma patients have significant levels of free lambda light chain in their circulation. As anti-LMA antibodies react with these free light chains, their presence in the fluid of the subject may reduce the efficiency of the treatment. Accordingly, in a preferred embodiment of the invention the method of treatment further comprises the step of treating the subject to reduce the levels of free lambda light chains circulating in the fluid (e.g. blood) of the subject prior to administration of the anti-LMA antibody. This additional treatment step may involve, for example, plasmapheresis. As will be known by those skilled in the art, plasmapheresis is a process in which the plasma is removed from blood cells by a device known as a cell separator. The separator works either by spinning the blood at high speed to separate the cells from the fluid or by passing the blood through a membrane with pores so small that only the plasma can pass through. The cells are returned to the subject, while the plasma, which contains the free kappa light chains, is discarded and replaced with other fluids. Medication to keep the blood from clotting (e.g. an anticoagulant) may be given through a vein during the procedure.

It will be appreciated that methods of treating B-cell disorders such as multiple myeloma involving the use of anti-LMA antibodies may be performed in isolation or as an adjunct to known chemotherapy or radiotherapy regimes. For example, anti-LMA antibody treatment may be conducted in conjunction with or after treatment with drugs such as melphalan or cyclophosphamide.

### Pharmaceutical Compositions, Dosages, and Routes of Administration

The present invention is also directed to pharmaceutical compositions comprising an anti-LMA antibody together with a pharmaceutically-acceptable carrier or diluent.

The antibodies and pharmaceutical compositions of the invention are useful for parenteral, topical, oral, or local administration, such as by aerosol or transdermally, for prophylactic and/or therapeutic treatment. The preferred route of administration of anti-LMA antibodies is parenteral; as used herein, the term "parenteral" includes intravenous, intramuscular, subcutaneous, rectal, vaginal or intraperitoneal administration. Of these, intravenous administration is most preferred.

The compositions for administration will commonly comprise a solution of the antibody dissolved in a pharmaceutically acceptable carrier, preferably an aqueous carrier. A variety of aqueous carriers can be used, e.g., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of antibody in these formulations can vary widely, and will be selected primarily based on fluid volumes, viscosities, body weight and the like in accordance with the particular mode of administration selected and the patient's needs.

The growth of tumour cells may be inhibited or reduced by administering to a subject in need of the treatment an effective amount of anti-LMA antibody. Typically, the antibody may be administered in an amount of about 0.001 to 2000 mg/kg body weight per dose, and more preferably about 0.01 to 500 mg/kg body weight per dose. Repeated doses may be administered as prescribed by the treating physician. However, other amounts are also suitable. Generally, the administration of the antibody is conducted by infusion so that the amount of antibody present that may produce a detrimental effect may be kept under control by varying the rate of administration. Typically, the infusion of one dose may last a few hours. However, also contemplated herein is the constant infusion of a dose for therapeutic purposes that will permit the maintenance of a constant level of the antibody in serum. The infusion of the anti-LMA antibody may be conducted as follows. Intravenous (I.V.) tubing may be pretreated, e.g., with 0.9% NaCl and 5% human serum albumin and placed for intravenous administration. The I.V. infusion may comprise a total volume of 250 ml of 0.9% NaCl and 5% human serum albumin and be infused over a period of about 2 hours depending on any rate-dependent side effects observed. Vital signs should be taken, for example, every fifteen minutes during the infusion and every one hour post infusion until stable. A thorough cardiopulmonary physical examination may be done prior to, and at the conclusion, of the infusion. Medications including acetaminophen, diphenhydramine, epinephrine, and corticosteroids may be kept at hand for treatment of allergic reactions should they occur. The administration of the antibody may be repeated as seen desirable by a practitioner.

In one example of the present invention, a radiolabeled form of the anti-lambda antibody is delivered by intravenous injection as a therapeutic agent to target cells that express the LMA. Previous examples of radiolabeled antibodies and the methods for their administration to patients as therapeutics are known to those skilled in the art. Examples include Iodine¹³¹ labeled Lym-1, against the β subunit of HLA-DR (DeNardo SJ et al. Antibody Immunoconj Radiophar (1988) 1:17-33; DeNardo SJ et al. Int J Biol Markers (1987) 2:49-53) and the anti-CD20 Indium¹¹¹ and Yttrium labeled Ibritumomab Tiuxetan (IDEC-Y2B8, ZEVALIN^{®}) and Iodine I 131 Tositumomab (BEXXAR^{®}).

In any treatment regimen, the therapeutic composition may be administered to a patient either singly or in a cocktail containing other therapeutic agents, compositions, or the like, including, but not limited to, immunosuppressive agents, tolerance-inducing agents, potentiators and side-effect relieving agents. Particularly preferred are immunosuppressive agents useful in suppressing allergic reactions of a host. Preferred immunosuppressive agents include prednisone, melphalain, prednisolone, DECADRON (Merck, Sharp & Dohme, West Point, Pa.), cyclophosphamide, cyclosporine, 6-mercaptopurine, methotrexate, azathioprine and i.v. gamma globulin or their combination. Preferred potentiators include monensin, ammonium chloride, perhexiline, verapamil, amantadine and chloroquine. All of these agents are administered in generally accepted efficacious dose ranges such as those disclosed in the Physician's Desk Reference, 41st Ed., Publisher Edward R. Barnhart, N.J. (1987), Patent Cooperation Treaty (PCT) patent application WO 89/069767 published on Aug. 10, 1989.

### Diagnostic Assays and Kits

The antibodies of the present invention can also be useful for diagnostic applications, both *in vitro* and *in vivo,* for the detection of human multiple myeloma cells. *In vitro* diagnostic methods include immunohistological detection of tumor cells. Immunohistochemical techniques involve staining a biological specimen such as a tissue specimen with the antibody of the invention and then detecting the presence of antibody complexed to its antigen as an antigen-antibody complex. The formation of such antibody-antigen complexes with the specimen indicates the presence of multiple myeloma cells in the tissue. Detection of the antibody on the specimen can be accomplished using techniques known in the art such as immunoenzymatic techniques, e.g., immunoperoxidase staining technique, or the avidin-biotin technique, or immunofluorescence techniques (see, e.g., Ciocca et al., "Immunohistochemical Techniques Using Monoclonal Antibodies", Methods Enzymol, 121:562-79, 1986 and Kimball, (ed), Introduction to Immunology (2.sub.nd Ed), pp. 113-117 (Macmillan Pub. Co., 1986).

For example, detection is by the detection of a label bound to the fusion polypeptide. Means of labeling polypeptides are well known to those of skill in the art. Labels may be directly linked through a covalent bond or covalently through a linking molecule which typically bears reactive sites capable of forming covalent bonds with the label and the antibody respectively, a common approach is to label the polypeptide and the label with either avidin or streptavidin or biotin which, in turn, bind irreversibly with each other.

Suitable labels are well known to those of skill in the art. The term "label", as used herein, refers to a composition detectable by spectroscopic, photochemical, biochemical, immunochemical, or chemical means. For example, useful labels include radioactive molecules such as ³²P, ¹⁴C, ¹²⁵I, ³H, and ³⁵S, fluorescent dyes such as fluorescein or rhodamine, electron-dense reagents, isothiocyanate; chromophores, enzymes (as commonly used in an ELISA), luminescent enzymes such as luciferase and the like.

Such labeled antibodies or binding fragments may be used for the histological localization of the antigens, for ELISA, for cell sorting, and for other immunological techniques to detect and/or quantify the antigens, and cells bearing the antigens, for example. As noted above, a particular use of such labeled antibodies, or fragments thereof, is in determining the effectiveness of myeloma cell depletion from bone marrow tissue prior to transplant, particularly autologous bone marrow transplant.

The present invention can be used for imaging methods for multiple myeloma using anti-LMA antibodies as described hereinabove. Other cancers bearing the LMA can also be amenable to these diagnostic procedures. The use can involve administration or infusion of monoclonal antibodies or binding fragments as described herein, with or without conjugation to a detectable moiety, such as a radionuclide. After administration or infusion, the antibody, or antibody fragment, binds to the tumor cells, after which the location of the antibodies, or fragments, is detected. For detectably-labeled antibodies or their binding fragments, such as those labeled with a radionuclide, imaging instrumentation may be used to identify the location of the agent within the body. For use of unlabeled antibodies or fragments, a second, detectable reagent may be administered which locates the antibodies or antibody fragments, and thus may be suitably detected. These methods have been used for other antibodies, and the skilled artisan will be amply aware of these various methods for imaging the location of antibodies or fragments within the body.

Detection of the anatomic location of LMA bearing cancer cells can be useful for the subsequent planning of antitumor therapy in each particular patient In particular, immunohistochemical pathologic diagnosis in tissue sections (e.g., biopsies), fluid samples (e.g., blood) or cytological preparations can be performed using the fusion polypeptides of the present invention.

This invention can be used for kits for research or diagnostic purposes. A kit typically includes one or more containers containing an anti-LMA antibody. The anti-LMA antibody may be derivatized with a label or, alternatively, it may be bound with a secondary label to provide subsequent detection. As described above, such labels may include radiolabels, fluorescent labels, enzymatic labels, i.e., horseradish peroxidase (HRP), or the like. The kit may also contain appropriate secondary labels (e.g., a sheep antimouse-HRP, or the like). The kit may also contain various reagents to facilitate the binding of the fusion polypeptides, the removal of non-specific binding antibodies, and the detection of the bound labels. Such reagents are well known to those of skill in the art.

In a further aspect of the present invention, compositions are provided which comprise the monoclonal antibody, or antibody binding fragment as described herein, bound to a solid support. A solid support for use in the present invention will be inert to the reaction conditions for binding. A solid phase support for use in the present invention must have reactive groups or activated groups in order to attach the monoclonal antibody or its binding partner thereto. In another embodiment, the solid phase support may be a useful chromatographic support, such as the carbohydrate polymers SEPHAROSB.RTM., SEPHADEX.RTM., or agarose. As used herein, a solid phase support is not limited to a specific type of support. Rather, a large number of supports are available and are known to one of ordinary skill in the art. Solid phase supports include, for example, silica gels, resins, derivatized plastic films, glass beads, cotton, plastic beads, alumina gels, magnetic beads, membranes (including, but not limited to, nitrocellulose, cellulose, nylon, and glass wool), plastic and glass dishes or wells, etc.

Methods for using the research and diagnostic kits described above are generally well known, and are generally provided in an instruction manual for use of the kit.

In order that the present invention may be more clearly understood preferred forms will be described with reference to the following non-limiting examples.

### EXAMPLES

### Materials and Methods

### Antibodies

The murine monoclonal antibodies (mAbs) used in these studies were raised against human free lambda light chains (λLCs) and were isotype IgG2a. The mAbs designated herein as L7 (clone 3D12) and ME 154 (clone ME-154) were obtained from AbCam Ltd. (Cambridge, UK). The mAb designated herein as mab1306 (clone HP6054) was obtained from Chemicon International Inc. (Melbourne, Victoria, Australia).

### Cell line

A human lambda-type multiple myeloma cell line (LP-1) was obtained from the German Collection of Microorganisms and Cell Cultures (DSMZ, Braunschweig, Germany). Cells were maintained in Iscoves MDM and 20% FBS at 37 °C with 5% CO₂ according to DSMZ recommendations.

### ELISA

The specificity of the mAbs L7, mab1306 and ME 154 for a variety of human free lambda light chains (λLCs) was established by enzyme-linked immunosorbent assay (ELISA). Specific antigens consisted of human free lambda light chains (Bence Jones Proteins, BJP) that were isolated from the urine of patient's with lambda type multiple myeloma. Four λLC samples, Lam F, Lam H, Lam K and Lam Q were purified and separated into monomer and dimer fractions using HPLC at the Australian Proteomic Analysis Facility (APAF). Lambda light chains associated with heavy chain were represented by intact polyclonal lambda-type human gammaglobulins (IgGλ) purified from normal human sera, obtained from Bethyl Laboratories, Inc. (Montgomery, Tx, USA). An irrelevant control antigen consisted of human free kappa light chains (κLC) in both monomer and dimer form.

The wells of an ELISA plate were coated with each antigen in phosphate buffered saline pH 7.4 with 0.02% sodium azide (PBS-az). After incubation at 37°C for 1 hour, the wells were washed three times with PBS-az and blocked with 3% BSA in PBS-az overnight at 4°C. The mAbs were incubated with antigen at 37°C for 3 hours and then the wells were washed three times with PBS-az. A goat anti-mouse IgG-AP conjugate (Sigma-Aldrich, St. Louis, MO, USA) was added to each well and incubated for 1 hour at 37°C. Finally the wells were washed as described above and the substrate (pNpp; Sigma) was added to each well. The colour development was proportional to the amount of mAb bound to the antigen, and was measured as absorbance at 405nm on an ELISA plate reader.

### Surface Plasmon Resonance (SPR)

The specificity of mAbs L7 and mab1306 for human free lambda light chains was confirmed using SPR on a Biacore 2000 biosensor instrument. MAbs L7 and mab1306 were immobilised on a dextran-coated Biacore CM5 chip via amine coupling, and the same Bence-Jones antigens as used in the ELISA were injected over the immobilised mAbs at 10µg/mL for 12 minutes. MAb-antigen binding was measured by SPR in Resonance Units, which was proportional to the mass increase at the chip surface caused by mAb capture of antigen.

### Flow Cytometry

Binding of the mAbs L7, mab1306 and ME 154 to the surface of LP-1 myeloma cells was evaluated by flow cytometry. LP-1 cells were harvested, washed by centrifugation and resuspended at a density of 1 x 10⁶ cells/mL in PBS-az with 1% BSA. Aliquots of 5 x 10⁵ cells were pelleted and then incubated with mAb (100 µg/mL) for 30 minutes on ice. The control samples consisted of an isotype (IgG2a) matched control mAb at the same concentration, or PBS-az with 1% BSA alone. After incubation with antibody, cells were washed twice in 750 µL of PBS-az with 1% BSA and incubated in 50 µL of a 1:20 dilution of PE-conjugated goat anti-mouse F(ab')₂ (Dako) for 30 minutes on ice. Cells were washed twice before analysis on the FL-2 channel of a FACSCalibur flow cytometer (BD Biosciences).

### Free λLC Inhibition of L7 Binding to LP-1 cells

L7 (100µg/mL) was pre-incubated with various concentrations of free lambda light chain dimers (Lam F and Lam H) or with κLC (800µg/mL) for 1 hour at 37°C. After pre-incubation the antibody mixtures were added to 5 x 10⁵ cells and binding was determined by flow cytometry as described above.

### Identification of a free λ LC antigen on the surface of LP-1 Cells by Fluorescence Microscopy

The localisation of immunoglobulin heavy (γ) and light (λ) chains was investigated by 2-colour fluorescence microscopy. The γ chain was detected using goat anti-human γ-specific antibodies conjugated to Fluorescein Isothiocyanate (FITC) and obtained from Open Biosystems (Huntsville, AL, USA). The λ chain was detected using goat anti-human λ antibodies conjugated to Texas Red and they were obtained from EY Laboratories Inc. (San Mateo, CA, USA). The anti-λ antibodies bound to both the free and heavy chain associated light chain. Antibody binding to LP-1 cells was demonstrated by incubating 10⁶ cells with 200µg/mL of anti-γ-FITC and anti-λ-Texas Red for 30 minutes on ice in PBS-az with 1% BSA. Cells were washed twice before analysis with a BX51 Fluorescence Microscope (Olympus, Tokyo, Japan). Location of surface immunoglobulin was detected under UV light using a 470-490nm bandpass excitation filter to show FITC staining (green). Location of surface λ light chain was detected under UV light using a 520-550nm bandpass excitation filter to show Texas Red staining (red). The FITC and Texas Red images were then overlayed to produce a two-colour image. Where FITC and Texas Red co-localise, the red and green colours merge to produce yellow. In this way, it is possible to determine whether λ light chains occur in regions other than those also occupied by γ chain.

### Identification of a free λ LC antigens in the cell membrane fraction of LP-1 cells by Western Blotting

The presence of a free λ LC antigen in the cell membrane fraction of LP-1 cells was demonstrated by Western blotting. LP-1 cells (4.5 x 10⁷) were washed twice by centrifugation with PBS and then resuspended at a concentration of 10⁷ cells/mL in 40 mM Tris pH 7.2 and complete protease inhibitors (Roche). The cells were vortexed for 30 seconds, sonicated for 15 mins and then vortexed again for 30 seconds. After incubation at room temperature for 20 minutes the cells were vortexed and then centrifuged at 4000g for 10 minutes. The cytoplasmic fraction was obtained from the supernatant and the cell membrane fraction consisted of the pellet. The pellet was washed twice by centrifugation with PBS and then resuspended in Tris buffered saline (TBS) containing 1% NP 40. The solubilized cell membrane was incubated for 30 minutes on ice and then centrifuged at 4000g for 10 minutes. The supernatant was collected as the solubilised cell membrane fraction. Aliquots of the cytoplasmic and cell membrane fractions were subjected to SDS-polyacrylamide gel electrophoresis using non denaturing conditions and then blotted onto nitrocellulose membranes. Detection of free λ LC antigen was detected by incubation with mAbs L7, mab1306 and ME 154 for 90 minutes at room temperature followed by 3 washes in TBS containing 0.05% Tween and 0.3% BSA. Membranes were then incubated with anti-mouse GAM-AP conjugate (Sigma) for 90 minutes and then washed 3 times as above. Colour development of the protein bands was performed by incubating the membranes with BCIP and NBT (Sigma *FAST™*)*.*

### Antibody Dependent Cell-mediated Cytotoxcitiy (ADCC)

The ability of mAbs mab1306 and ME 154 to induce ADCC was assessed *in vitro* using the flow cytometric method of Wilkinson et al (Journal of Immunological Methods 2001 258: pp183-91). Natural Killer (NK) cells were used as effector cells and LP-1 myeloma cells were used as the targets.

Mouse NK cells were isolated from a suspension of splenic lymphocytes harvested from healthy Balb/c mice using MACS Magnetic Cell Sorting (Miltenyi Biotech, Germany). Using the Miltenyi Biotech NK Cell Isolation Kit, all lymphocytes other than NK cells were removed, leaving purified, unlabelled NK cells. The NK cells were cultured for 6 days in RPMI 10% FCS supplemented with 125ng/mL of recombinant murine IL-2 (Sigma-Aldrich, USA).

Assays were carried out in U-bottomed 96-well tissue culture plates (Nunc, Denmark). Immediately prior to the assay, target cells (LP-1 myeloma cells) were labelled with the fluorescent membrane dye PKH-26 (Sigma-Aldrich, USA) according to manufacturer's instructions. LP-1 cells (3x10⁴cells/mL) were pre-incubated with either ME 154 or mab1306 in sterile PBS (7nM and 0.7nM final concentration) for 15 minutes at 37°C and 5% CO₂. Effector cells, murine NK cells washed and resuspended in RPMI, were then added to give NK to LP-1 ratios of 25:1, 12.5:1 and 6.25:1. Cells were mixed and then the plates were centrifuged for 10 minutes at 400g to ensure close cell-cell contact. Plates were incubated at 37°C for 16 hours.

After 16 hours, 50µL of the DNA binding dye TO-PRO-Iodide 3 (1µM in PBS; Molecular Probes Inc., USA) was added to each test sample. The TO-PRO-Iodide 3 can only enter cells with compromised membranes, wherein it binds to double stranded DNA. Only upon such DNA binding does the TO-PRO-Iodide 3 fluoresce. After 2-5 minutes the cells were run on a FACSCalibur flow cytometer (BD Inc., USA). Dead cells were identified by their positive fluorescence in the FL4 channel caused by TO-PRO-Iodide 3/DNA complexes. Target (LP-1) cells were identified by positive fluorescence in the FL2 channel caused by PKH-26 labelling. Dead target cells were therefore identified as those of positive fluorescence in both the FL2 and FL4 channels. Percentage cytotoxicity was calculated as: (number of FL4- FL2 double positive cells)/(number of FL2 positive cells)*100.

### Example 1: Specificity of the antibodies L7, mab 1306 and ME 154 for λ light chain.

The specificity of L7 binding to human free lambda light chains is shown in Figures 1A and 2A. These results indicate that L7 binds to both the monomer and dimer forms of three different lambda light chains (Lam F, Lam H and Lam K), and to the monomeric form of a fourth lambda light chain (Lam Q). The antibody does not bind to lambda light chains associated with heavy chain in normal human immunoglobulin, nor does it bind free kappa light chains.

The antigen binding properties of mab1306 to a range of human lambda light chains is shown in Figures 1B and 2B. These results demonstrate that mab1306 binds to a range of free and immunoglobulin-associated λ. LCs but does not bind to free κ LCs. Similarly, mAb ME 154 binds to both free and heavy chain associated λ LCs (Figure 1C). All three mAbs show binding to the monomeric form of Lam Q but not to the dimer form of this antigen.

### Example 2: Identification of λ, light chains on LP-1 myeloma cells.

Flow cytometry results indicate that L7, mab1306 and ME 154 all bind specifically to a cell surface antigen on LP-1 myeloma cells (Figure 3). For mAb L7, antibody binding to LP-1 cells is inhibited by pre-incubation with two different monomeric free lambda light chains, Lam F and Lam H (Figure 4). This inhibition occurs in a concentration-dependent manner (Figure 4B), however L7 binding is not inhibited by an equivalent concentration of free kappa light chain.

The presence of a free λ light chain antigen that is not associated with Ig heavy chain was demonstrated on the surface of LP-1 cells using fluorescence microscopy (data not shown). Incubation of the cells with fluorescently labelled polyclonal antibody specific for the IgG gamma chain (anti-γ-FITC) showed non-uniform intensity of green staining of LP-1 cells, whereas polyclonal antibody against λ light chains, anti-λ-Texas Red, showed several patches of intense red on the surface of the cells (data not shown). The most intense patches of red colour (λ light chains) appeared to be in different locations to the green colour (Ig associated γ chains). This observation was confirmed by overlaying the two colour pictures of the same cell (data not shown). A combined red and green colour produced yellow, whereas an area that was stained with only one of the antibodies retained the original colour. The yellow colour on the cell surface represented λ light chain that was associated with immunoglobulin. However, there were distinct patches of red colour that indicated patches of λ light chain that were not associated with Ig and are therefore areas of free λ light chain on the cell surface.

Detection of membrane-associated λ light chain from LP-1 myeloma cells was carried out by western blot of non-reducing SDS-PAGE using mAbs L7, mab1306 and ME 154 (Figure 5). A positive control consisted of a purified human free λ light chain which was detected by all three antibodies. Both mab 1306 (Figure 5B) and ME 154 (Figure 5C) detected the monomer (25 kD) and dimer (50 kD) forms of free λ light chain in the membrane and cytoplasmic fractions of the cells. MAb L7 showed binding to the membrane associated monomer and dimer forms of free λ light chain but appeared to detect only the monomer form of the antigen in the cytoplasm.

### Discussion

The murine monoclonal antibody, L7, binds specifically to four different human free lambda light chains and does not bind to lambda light chains associated with heavy chain. Analysis of antibody binding to the lambda type myeloma cell line, LP-1, indicates that the antibody binds to a cell surface antigen. Binding to the cell surface antigen can be blocked by pre-incubating the antibody with two different lambda light chains. In addition, the soluble form of free lambda light chain can completely abrogate binding of L7 to the cell surface antigen on LP-1 cells. These data suggest that the antibody recognises a cell surface antigen that contains similar epitopes found on free lambda light chains.

Fluorescence microscopy indicated that cell surface free λ light chain could be distinguished from Ig associated λ light chain on the surface of lambda type myeloma cells. In addition the detection of cell membrane free λ light chain in the form of a 25 kD monomer and 50 kD dimer could be demonstrated on the cell membrane fraction of lambda myeloma cells by all three mAbs.

Specific antigen dependent cellular cytotoxicity of lambda myeloma cells could be demonstrated using antibodies mab 1306 and ME 154. These results suggest that antibodies that bind to free λ light chain in the form of monomer and dimer on the surface of the cell can induce cell death in the presence of effector cells.

The experiments detailed herein demonstrate that several murine monoclonal antibodies against free lambda light chains can detect both the monomer (25kD) and dimer (50 kD) forms of the lambda antigen on the cell membrane. We propose that this LMA consists of free lambda light chains associated with the cell membrane and can be used to specifically target myeloma cells from patients with lambda type multiple myeloma using monoclonal antibodies.

In addition it is envisaged that the LMA is present on B cells from various B-cell disorders. Examples of such cells are DOHH-2 (human B cell lymphoma), WSU-NHL (human B cell lymphoma), DB (human B cell lymphoma), KARPAS-1106P (human B cell lymphoma), WSU-DLCL2 (human B cell lymphoma), SU-DHL-5 (human B cell lymphoma), MHH-PREB-1 (human B cell lymphoma) GRANTA-519 (human B cell lymphoma) and MC-116 (human Bcell lymphoma).

As LMA is unique to the cell membrane of the malignant B-cell it is proposed that any mAb that is capable of selectively binding these antigens will be useful in the treatment of diseases such as multiple myeloma. As a secondary effect any mAb of the appropriate isotype that is capable of binding LMA will be able to induce cell death by using the host effector cells to bring about ADCC. This secondary effect will aid the depletion of B-cells in patients with B-cell disorders.

Various modifications and variations of the described methods and system of the invention will be apparent to those skilled in the art. Although the invention has been described in connection with specific preferred embodiments, it should be understood that the invention as claimed should not be unduly limited to such specific embodiments. Indeed, various modifications of the described modes for carrying out the invention which are apparent to those skilled in molecular biology or related fields are intended to be within the scope of the invention.

## Claims

1. Use of an anti-lambda myeloma antigen antibody that binds specifically to lambda myeloma antigen in the preparation of a medicament for the treatment or prophylaxis of a B-cell lymphoproliferative disorder in a subject, wherein the B-cell disorder is multiple myeloma, and wherein lambda myeloma antigen is a lambda light chain not associated with an intact immunoglobulin and is expressed on the surface of myeloma cells.

2. A use as claimed in claim 1, wherein the anti-lambda myeloma antigen antibody is conjugated to a cytotoxic moiety or biological modifier.

3. A use as claimed in any one of claims 1 to 2, wherein the subject has been treated to reduce the level of free lambda light chains present in the fluid of the subject prior to administration of the anti-lambda myeloma antigen antibody.

4. An anti-lambda myeloma antigen antibody that binds specifically to lambda myeloma antigen for use in treating a B-cell lymphoproliferative disorder wherein the B-cell disorder is multiple myeloma, and wherein lambda myeloma antigen is a lambda light chain not associated with an intact immunoglobulin and is expressed on the surface of myeloma cells.

5. The anti-lambda myeloma antigen antibody as claimed in claim 4, wherein the anti-lambda myeloma antigen antibody is conjugated to a cytotoxic moiety or biological modifier.

6. The anti-lambda myeloma antigen antibody as claimed in any one of claims 4 to 5, wherein the subject has been treated to reduce the level of free lambda light chains present in the fluid of the subject prior to administration of the anti-lambda myeloma antigen antibody.

## Patentansprüche

1. Verwendung eines anti-lambda-Myelom-Antigen-Antikörpers, der spezifisch an lambda-Myelom-Antigen bindet, bei der Herstellung eines Arzneimittels zur Behandlung oder Prophylaxe einer lymphoproliferativen B-Zellstörung bei einem Subjekt, wobei es sich bei der B-Zellstörung um ein multiples Myelom handelt und wobei es sich beim lambda-Myelom-Antigen um eine lambda-Leichtkette handelt, die nicht mit einem intakten Immunoglobulin assoziiert ist und die an der Oberfläche von Myelomzellen exprimiert wird.

2. Verwendung nach Anspruch 1, wobei der anti-lambda-Myelom-Antigen-Antikörper mit einem zytotoxischen Rest oder einem biologischen Modifikator konjugiert ist.

3. Verwendung nach einem der Ansprüche 1 bis 2, wobei das Subjekt behandelt worden ist, um die Konzentration an freien lambda-Leichtketten, die im Fluid des Subjekts vor der Verabreichung des anti-lambda-Myelom-Antigen-Antikörpers vorhanden sind, zu verringern.

4. Anti-lambda-Myelom-Antigen-Antikörper, der spezifisch an lambda-Myelom-Antigen bindet, zur Verwendung bei der Behandlung einer lymphoproliferativen B-Zellstörung, wobei es sich bei der B-Zellstörung um ein multiples Myelom handelt und wobei es sich beim lambda-Myelom-Antigen um eine lambda-Leichtkette handelt, die nicht mit einem intakten Immunoglobulin assoziiert ist und die an der Oberfläche von Myelomzellen exprimiert wird.

5. Anti-lambda-Myelom-Antigen-Antikörper nach Anspruch 4, wobei der anti-lambda-Myelom-Antigen-Antikörper mit einem zytotoxischen Rest oder einem biologischen Modifikator konjugiert ist.

6. Anti-lambda-Myelom-Antigen-Antikörper nach einem der Ansprüche 4 bis 5, wobei das Subjekt behandelt worden ist, um die Konzentration an freien lambda-Leichtketten, die im Fluid des Subjekts vor der Verabreichung des anti-lambda-Myelom-Antigen-Antikörpers vorhanden sind, zu verringern.

## Revendications

1. Utilisation d'un anticorps d'antigène de myélome anti-lambda qui se lie de manière spécifique à un antigène de myélome lambda dans la préparation d'un médicament pour le traitement ou la prophylaxie d'un désordre lympho-prolifératif de cellules B chez un patient, dans lequel le désordre de cellules B est un myélome multiple, et dans lequel l'antigène de myélome lambda est une chaîne légère lambda non associée à de l'immunoglobuline intacte et est exprimé sur la surface des cellules de myélome.

2. Utilisation selon la revendication 1, dans laquelle l'anticorps d'antigène de myélome anti-lambda est conjugué à une fraction cytotoxique ou à un modificateur biologique.

3. Utilisation selon l'une quelconque des revendications 1 à 2, dans laquelle le patient a été traité afin de réduire le niveau des chaînes légères lambda libres présentes dans le fluide du patient avant l'administration de l'anticorps d'antigène de myélome anti-lambda.

4. Anticorps d'antigène de myélome anti-lambda qui se lie de manière spécifique à l'antigène de myélome lambda en vue d'une utilisation pour le traitement d'un désordre lympho-prolifératif de cellules B dans lequel le désordre de cellules B est un myélome multiple et dans lequel l'antigène de myélome lambda est une chaîne légère lambda non associée à de l'immunoglobuline intacte et est exprimé sur la surface des cellules de myélome.

5. Anticorps d'antigène de myélome anti-lambda selon la revendication 4, dans lequel l'anticorps d'antigène de myélome anti-lambda est conjugué à une fraction cytotoxique ou un modificateur biologique.

6. Anticorps d'antigène de myélome anti-lambda selon l'une quelconque des revendications 4 à 5, dans lequel le patient a été traité afin de réduire le niveau des chaînes légères lambda libres présentes dans le fluide du patient avant l'administration de l'anticorps d'antigène de myélome anti-lambda.
